# EUROPEAN PATENT SPECIFICATION

(11) **EP 1 087 935 B1**
(45) Date of publication and mention of the grant of the patent: **18.08.2004**
(21) Application number: 99926616.6
(22) Date of filing: 16.06.1999
(51) Int. Cl.: C07C 229/08, C07C 229/36, C07D 207/16, C07D 209/20, A61K 31/195, A61K 31/40, C07K 5/06, C07C 229/26

(54) **ANTHRACENE DERIVATIVES AS ANTI-CANCER AGENTS**
ANTHRACENE DERIVATIVE ALS ANTITUMORMITTEL
DERIVES D'ANTHRACENE UTILISES COMME AGENTS ANTICANCEREUX

(30) Priority: 16.06.1998 GB 9812937
(43) Date of publication of application: 04.04.2001
(73) Proprietor: BTG INTERNATIONAL LIMITED, London EC4M 7SB (GB)
(72) Inventor: MINCHER, David John, East Lothian EH42 1YD (GB); TURNBULL, Agnes, Edinburgh EH12 8XD (GB)
(74) Representative: Dolan, Anthony Patrick
(86) International application number: PCT/GB1999/001901
(87) International publication number: WO 1999/065866

(56) References cited:
- WO-A-93/19037
- WO-A-95/09149
- MASAYUKI ENDO ET AL: "RNA hydrolysis by the cooperation of carboxylate ion and ammonium ion" JOURNAL OF THE AMERICAN CHEMICAL SOCIETY, vol. 118, no. 23, 12 June 1996 (1996-06-12), page 5478/5479 XP002081307 ISSN: 0002-7863

## Description

The present invention relates to compounds which are based on an anthraquinone nucleus for use in medicine, particularly as anti-cancer agents which exert their effects through their interaction with the activity of topoisomerases.

The inhibition of DNA topoisomerases, particularly topoisomerase II (topo II) is now considered to be an important component in the mechanism of action of a large number of the most clinically active anticancer drugs presently available including doxorubicin, mitoxantrone, VP16, camptothecin, topotecan, M-AMSA, VM26 and the ellipiticines. These drugs are believed to inhibit topo II by stabilising a protein/drug/nucleic acid ternary complex termed the cleavable complex.

However, whilst targeting topoisomerases, the aforesaid prior art drugs also exhibit a number of other mechanisms of action, such as generation of free radicals and formation of DNA covalent adducts which contribute to their overall toxicity and poor therapeutic index. Additionally, the failure of these agents to produce long term cures in the major malignancies is probably exacerbated by the presence of *de novo* resistance and the development of acquired drug resistance.

US-A-4 894 451 describes asymmetrically substituted anthracene-1,4-dione compounds of Formula (A): 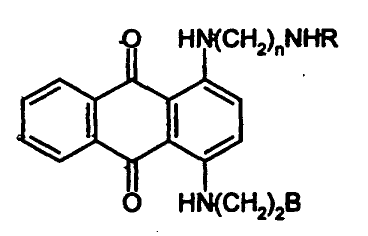 where B is a lower dialkyl amino group, n is 3-5 and R is hydrogen, alkanoyl or alkylsulphonyl. These compounds were proposed for use against tumours.

WO 93/19037 describes-compounds having the structural formula: 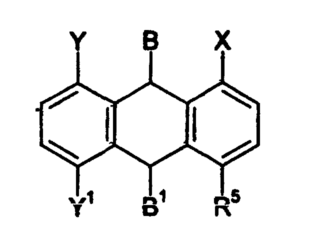 where Y and Y¹ are independently hydrogen or hydroxyl, B and B¹ are independently oxo or hydrogen, R⁵ is hydrogen or hydroxyl and X is the residue of an α amino acid or a derivative of an α amino acid, joined to the ring shown via the nitrogen atom of the amino acid adjacent the acid group thereof. These compounds provide clinically active drugs and coloured compounds useful as drugs.

EP-A-0 295 316 discloses symmetrically-substituted compounds for use in anti-tumour therapy, namely 1,4-bis[(aminoalkyl- and hydroxyaminoalkyl)-amino]-5,8-dihydroxyanthraquinones.

A series of papers from Morier-Teissier *et al* [(1989) *Anti-Cancer Drug Design* **4**, 37-52; ibid. (1990), **5**, 291-305; (1993) *J Med Chem* **36**, 2084-2090] disclosed various copper-chelating asymmetric peptide-anthraquinone compounds using a Gly-His-Lys or Gly-Gly-His moiety, or sometimes just the initial Gly, attached directly to the 4-position of the anthraquinone ring, with the 1-position being substituted by a hydroxyl group. It also describes bisubstitution by Gly-Gly-His. JP-A-82141456 describes N-[4-[(9,10-dihydro-9,10-dioxo-1-anthrasenyl)amino] carbonyl]benzoyl]-D,L-alanine as a dyestuff.

US 5733880 and its corresponding EP 0721447 disclose compounds of general formula 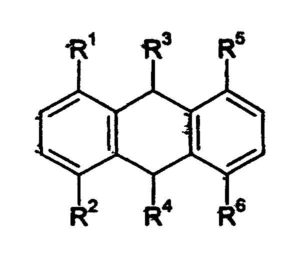 wherein R¹ and R² are independently hydrogen or hydroxyl, R³ and R⁴ are independently oxo or hydrogen, one of R⁵ and R⁶ is A-B and the other is hydrogen, hydroxyl or a group A, wherein the or each A is independently a spacer group providing NH or CO in the bond with B, if present, at least one group A does not provide the residue of an α-amino acid adjacent the anthraquinone nucleus and the A of any A-B moiety is joined to the anthraquinone nucleus via an -NH- bond, and the or each B is a peptide group or a physiologically acceptable derivative thereof. These compounds are described as being particularly useful antitumour compounds acting via topoisomerases and also to be useful as dyes.

It is an object of this invention to provide improved clinically active anticancer agents preferably having one or more of the following properties: (i) reduced or eliminated capacity to induce generation of free radical species, (ii) reduced or eliminated capacity to bind to DNA or RNA, (iii) different relative activity against topoisomerases I, IIα and IIβ as compared to the known prior art compounds and (iv) activity, particularly being improved as compared to known compounds of this type, against drug resistant cell lines.

Different activity as indicated in (iii) is advantageously relatively high Topoisomerase I and/or IIβ activity with respect to Topoisomerase IIα activity. Particularly preferred compounds may work through Topoisomerase I and/or IIβ, but not IIα.

According to a first aspect of the invention there is provided a compound having the Formula I: 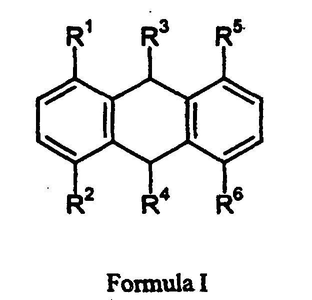 wherein
R¹ and R² are independently hydrogen, hydroxy, alkoxy or acyloxy;
R³ and R⁴ are independently oxo, hydroxy or hydrogen;
one of R⁵ or R⁶ is -A-B and the other is hydrogen, hydroxy, alkoxy, acyloxy, a group -A-B or a group -amino-R⁷-O-Y;
the or each A is independently a spacer group having the formula -amino-R⁷-O- which is bonded to the anthracene ring via the amino group nitrogen and to B via the -O- atom through an ester bond;
R⁷ is a divalent organic radical;
B is an amino acid residue or a peptide group or isostere thereof; and
Y is hydrogen or a capping group,
or a physiologically acceptable derivative of such compound.

Preferably R¹ and R² are independently selected from hydrogen and hydroxy, but when they are alkoxy or acyloxy, these are preferably selected from C₁₋₆ alkoxy or acyloxy, such as methoxy and ethoxy, or acetoxy and propionyloxy.

Clearly, when R³ or R⁴ are oxo, the single line to the ring represents a double bond. Preferred compounds are those of Formula II

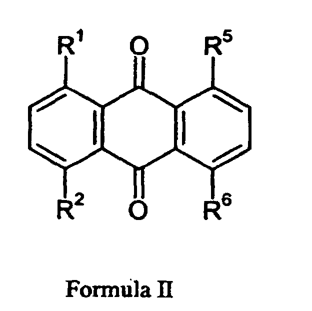

Preferably only one of R⁵ and R⁶ is a group -A-B and the other is hydrogen, hydroxy, alkoxy, acyloxy, more preferably hydrogen or hydroxy.

The term amino, as used with respect to -amino-R⁷-O-, may be a group -NH-, -NR¹⁰- or -N=R¹¹-. R¹⁰ is selected from alkyl, alkenyl, aralkyl or aryl, most preferably being alkyl. All R¹⁰ groups alkyl, alkenyl, aralkyl or aryl preferably contain only one or two C₁₋₆ alkyl groups and/or a single phenyl ring as appropriate. When amino is -N=R¹¹, R¹¹ consists of a moiety with which the -N= makes up a heterocylic ring system, preferably a single heterocyclic ring, containing the nitrogen atom of the aforesaid -N= moiety and up to 6, but preferably only 3, 4 or 5 other members selected from nitrogen, oxygen, sulphur and carbon. Most preferably such amino group is a ring selected from NC₄, NC₅, N₂C₃ and N₂C₄ rings, ie. pyrrole, 2H-pyrrole, pyrrolidine, pyrroline, imidazole, imidazolidine, imidazoline, pyrazole, pyrazolidine, pyrazoline, pyridine, pyrazine, piperidine, and piperazine. -R⁷- may be bonded to any of the atoms of the moiety completing the ring.

Preferably the or each A is independently a spacer group having the formula -NH-R⁷-O- which is bonded to the anthracene nucleus via the -NH- moiety and to B via the -O- moiety. Preferably one A only is linked to B. Preferably one of R⁵ and R⁶ is hydrogen or hydroxy.

R⁷ may be any divalent group that spaces the moiety -O- from the amino group on the anthracine ring system by a contiguous chain of 1 to 20 atoms, more preferably 1 to 12 atoms, and most preferably 2 to 6 atoms especially 3, 4 or 5 atoms. This group may consist of or include straight or branched alkylene chains which may be interrupted by one or more carbocylic or heterocyclic rings. These rings may be saturated or unsaturated. The alkylene chain may alternatively or additionally be interrupted by an olefinic bond.

Preferably R⁷ is an alkylene radical having the formula -(CHR)ₙ- where n is an integer, preferably of at least 2, and the or each R is independently hydrogen or an alkyl group. Preferably R is hydrogen. Suitably n is 2 to 20 and is preferably 2 to 12, more preferably 2 to 6. Thus, suitable alkylene radicals for R⁷ include ethylene, trimethylene, tetramethylene, pentamethylene and hexamethylene.

Other examples of groups R⁷ include those where the chain of carbon atoms of the aforesaid alkylene radical are interrupted by olefinic bonds, heteroatoms carbocylic and/or heterocyclic rings. For example, one or more of the methylene - CH₂- groups may be isoelectronically replaced by -O-, -NH- or -S-. It will be realised that the -NH- may require protection eg. by Boc, during syhthesis of the compounds of the invention, depending on synthetic route.

R⁷ may be branched, eg, by way of substituents on the alkylene chain such as halo, hydroxy, C₁₋₆ alkyl, C₁₋₆ hydroxyalkyl, C₁₋₆ alkoxy, C₇₋₁₂aralkyl, eg benzyl. Further examples include groups including chiral centre carbons in the chain, eg. where the alkylene chain is substituted with alkyl such as in -NH-CH(C₂H₅)-(CH₂)₂-O-, where R⁷ is -CH(C₂H₅)-(CH₂)₂-, and gem-dialkyl groups centred on chain carbon atoms, such as in -NH-C(CH₃)₂-(CH₂)₂-O-where R⁷ is C(CH₃)₂-(CH₂)₂-.

B may be a single amino acid residue, an oligopeptide or a polypeptide. Where it is an oligopeptide it is typically of no more than 100 amino acid residues, eg. no more than 50, but more preferably from 1 to 10 amino acids and especially, eg. di, tri, tetra or penta-peptide. Most conveniently B is a single amino acid residue. The peptide group may contain spacer groups between the amino acids thereof. If present, such spacer groups are preferably selected from the same possibilities as group A and may alternate with the amino acid residues or otherwise replace all but key amino acids in a recognition sequence. B is preferably an α-amino acid or a peptide group made from α-amino acids. By "α amino acid", we mean a compound such as those specified in US 5,733,880, column 3, line 55 to column 4, line 39.

The di-, tri-, tetra-, penta-, oligo and polypeptides may be of any suitable amino acid sequence. One possible sequence (Suzuki (1989) *EMBO J.* 8, 797). (Ser-Pro-Lys-Lys)ₙ wherein n is 1 to 10, has been proposed as discussed in US 5,733,880 and EP 0721447. Useful intermediates for synthesising such peptides are described in US 5,733,880 column 4, lines 45 to 65. The syntheses of these compounds are described in detail in Bailly *et al* (1992) *Anti-Cancer Drug Design* (1992) 7, 83-100 incorporated herein by reference wherein the peptide was joined to the acridine heterocyclic ring system at the position opposite the N heteroatom in the middle ring.

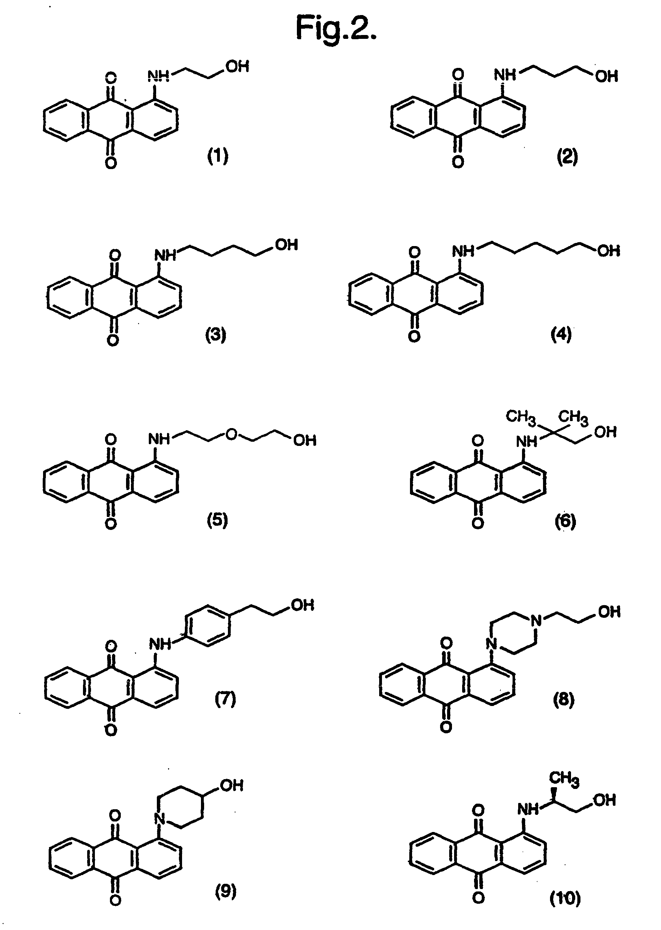

By "isosteres" of the amino acids or peptides we include ώ-amino acids that have side chains that mimic the characteristic side chains of α-amino acid and peptides used in the invention. Examples of conventional isosteres are illustrated in 'A Practical Guide to Combinatorial Chemistry, (1997) Edits. Czarnik and DeWitt, American Chemical Society ISBN 0-8412-3485-X, page 57, Figure 2, eg. depsipeptides and peptoids, wherein the sidechains characteristic of α-amino acids are in alternative carried on ester group carbons or on amide group nitrogens; and in Medicinal Chemistry: Principles and Practice (1998) Edit: F D King, The Royal Society of Chemistry, ISBN-0-85186-494-5, Chapter 14, see Tables 2 page 208 re carboxylic amide groups in peptides; both incorporated herein by reference Also included are peptide mimics corresponding to peptides with amide bonds replaced by olefinic bonds.

By "derivatives" of the compounds of the invention, we include salts (acid or base addition), esters, amides, hydrazides and hydroxamic acids of the group B and other derivatives which do not diminish to an unacceptable extent the fundamental topoisomerase mediated activity, ie. anti-tumour properties of the compounds.

Salts which may be conveniently used in therapy include physiologically acceptable base salts, for example, derived from an appropriate base, such as an alkali metal (e.g. sodium), alkaline earth metal (e.g. magnesium) salts, ammonium and NX₄⁺ (wherein X is C₁₋₄ alkyl) salts. Physiologically acceptable acid salts include hydrochloride, sulphate, trifluoroacetate, mesylate, besylate, phosphate and glutamate.

Salts according to the invention may be prepared in conventional manner, for example by reaction of the parent compound with an appropriate base to form the corresponding base salt, or with an appropriate acid to form the corresponding acid salt.

Further preferred derivatives include those in which functional groups on the peptide group, which may be side groups or the terminal group, are capped. Suitable chemical groups to cap -NH- include -COCH₃, tertiary-butoxycarbonyl, benzyloxycarbonyl and other groups known in the art. Suitable chemical groups to cap -CO- include -OH or any -O-linked or -N-linked radical, for example -O-alkyl,-O-benzyl, -O-alkylaminoalkyl, -O-alkoxyalkyl or -NH-NHR⁹ where R⁹ is straight or branched alkyl, optionally substituted by -CN or -OH, an amide group (such as-CONH₂) and other groups known in the art. Examples of alkylaminoalkyl groups include CH₃(CH₃)NCH₂CH₂-, -(CH₂)₂NH(CH₂)₂OH and CH₃(CH₃)NCH₂CH₂NHCH₂CH₂-. Preferred capped -NH- groups are those where the side chain is capped as opposed to the terminal -NH₂.

Where not otherwise defined, by "alkyl", we include branched or straight chain alkyl of up to 20 carbon atoms, preferably 1-10 carbon atoms, more preferably 1-6 or 1-4 carbon atoms.

A useful discussion of alternative protective groups for amino acids (all types) and the scope of coupling reagents and deprotection reactions is to be found on pages 153-184 of a section called "chemical synthesis of peptides" in chapter 3 "Amino acids and Peptides" by R.S. Davidson and J.B. Hobbs in: "Natural Products, their Chemistry and Biological Significance", Authors: J. Mann, R.S. Davidson, J.R. Hobbs, D.V. Banthorpe & J.B. Harbome, publ. Longman Scientific and Technical (1994).

It has been found that the compounds of the invention may be prepared as substantially pure optical isomers, ie. it is possible to synthesise them without inducing racemisation of chiral groups.

B is preferably the residue of an amino acid or oligopeptide and conveniently is the residue of an α-amino acid, but may be β-, γ-, δ-, ε-, ζ-, η- amino acids where these are isosteres of peptides comprised of α-amino acids. For the avoidance of doubt, by the residue of an amino acid we mean the group which would remain after the carboxyl (-C(O)O-) functionality on the original amino acid has reacted to bond the amino acid to the -O-group of amino-R⁷-O-, by way of an ester bond, at the distal end of the spacer group A to the anthracene ring moiety and in so doing become incorporated into the spacer group A, or a salt thereof. Thus, when B is the residue of an α-amino acid having the formula given above, it will have the formula:

HₙN⁺-CHR⁸-C(O)- or [X⁻] HₘN⁺-CHR⁸-C(O)-

where R⁸ is a characteristic group of such acid, eg such as specified as in US 5,733,880 for R⁷, and n is 1 or 2 m is 2 or 3 and X⁻ is a counter ion.

Preferably B comprises one or more independently selected residues of alanine, phenylalanine, glycine, proline, valine, leucine, methionine or tyrosine. Particularly preferred residues are of amino acids and peptides the internal amide bonds or the ester bond to -amino-R⁷-O- of which that are resistant to degradation by enzymes *in vivo*. For example use of D-amiao acids or N-alkylated amino acids such as N-methylglycine (sar), N-methylalanine. More preferred are di-, tri- and tetrapeptides. The L-isomer is usually preferred in each case, although D-isomers may be preferred, eg D-Phe.

Preferred groups Y include a hydrogen atom and alkyl, aryl, aralkyl, e.g. benzyl, and acyl, e.g. tert-butoxy-carbonyl, groups.

In one preferred embodiment, R¹ and R² are both H, R³ and R⁴ are both oxo, R⁵ is a group -A-B and R⁶ is H. In another preferred embodiment, R¹ is OH, R² is H, R³ and R⁴ are both oxo, R⁵ is a group -A-B and R⁶ is OH. In yet another preferred embodiment, R¹ and R² are both H, R³ and R⁴ are both oxo, R⁵ is a group -A-B and R⁶ is OH. In a further preferred embodiment, R¹ and R² are both OH, R³ and R⁴ are both oxo, R⁵ is a group -A-B and R⁶ is OH.

A further aspect of the invention provides a process for preparing a compound of formula I comprising:
(A) reacting a compound of Formula IV 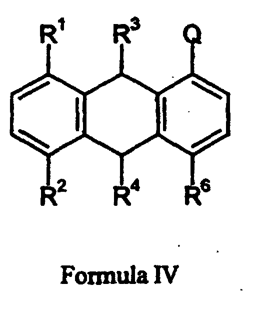 where R¹ to R⁴ are as defined above, R⁶ is hydrogen or hydroxyl and Q is a reactive group such as -Cl, -Br or -OH with an amino alcohol, e.g. an ω-aminoalkanol, to form a compound having the formula V: 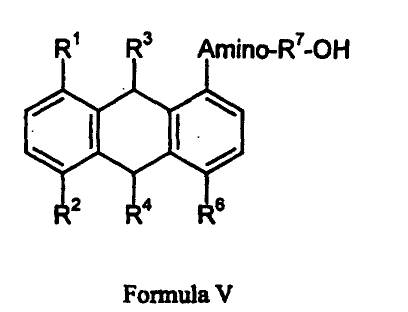
and (B) reacting the compound of Formula V with an amino acid or peptide or isostere to give a compound of Formula I.

Compounds of Formula IV in which Q is Cl or Br and both R³ and R⁴ are oxo are commercially available. The reaction generally proceeds in an aprotic solvent (e.g. DMSO or DMF). One compound of the invention can be converted to another by, for example, oxidising -H at R¹ and/or R² to -OH; oxidising -H at R³ and/or R⁴ to -OH; oxidising -OH at R³ and/or R⁴ to oxo, for example in an aerial oxidation or using chloranil; or reducing oxo at R³ and/or R⁴ to -OH (for example with sodium dithionite or zinc/acetic acid) or onward to -H. The sodium dithionite reaction is described in Marschalk *et al* (1936) *Bull. Soc. Chim. Fr*. **3**, 1545, and the Zn/CH₃COOH reaction in Morris, G.A. *et al* (1986) *Tetrahedron* **42**, 3303 Another conversion of one compound of the invention to another involves extending the B group by removing any cap which is present and adding one or more amino acid residues.

Prior to step (B), the amino group of the amino acid should be protected by a group such as tertiary-butyloxycarbonyl, benzyloxycarbonyl, fluorenylmethoxycarbonyl, and the like, to avoid interference during condensation with the anthracene compound eg. the anthraquinone. Similarly, those amino acids which contain functionality in their side-chains in general also need to have the functionality protected. The protecting groups used on the side chain can be the same or different than those used to protect the amino radical. The protecting group can be removed after step (B) has been completed. Methods for applying and removing protecting groups are taught in US 5,733,880, column 9, line 9 to column 10, line 20.

In a further aspect the invention provides a pharmaceutical preparation comprising a pharmaceutically acceptable carrier and/or excipient and a compound of the first aspect. Any suitable pharmaceutically acceptable carrier can be used. The preparation should be suitable for administration in the chosen manner. In particular, it should be sterile and, if intended for infection, non-pyrogenic.

Administration of the aforementioned compounds of the invention or a formulation thereof need not be restricted by route. Options include enteral (for example oral and rectal) or parenteral (for example delivery into the nose or lung or injection into the veins, arteries, brain, spine, bladder, peritoneum, muscles or subcutaneous region. The compounds may be injected directly into the tumour. The treatment may consist of a single dose or a plurality of doses over a period of time. The dosage will preferably be determined by the physician but may be between 0.01 mg and 1.0 g/kg/day, for example between 0.1 and 500 mg/kg/day. In terms of dose per square meter of body surface, the compound can be administered at 1.0 mg to 1.5 g per m² per day, for example 3.0-200.0 mg/m²/day. At least some compounds of the invention have a particularly low toxicity to normal mammalian cells and could be given in quite high doses, for example 50-300 mg/kg. By comparison doxorubicin has a maximum tolerated dose of 5 mg/kg in rodents and 1-2 mg/kg in man.

Whilst it is possible for a compound of the invention to be administered alone, it is preferable to present it as a pharmaceutical formulation, together with one or more acceptable carriers and/or excipients. The carrier(s) and/or excipients must be "acceptable" in the sense of being compatible with the compound of the invention and not deleterious to the recipients thereof.

The formulations may conveniently be presented in unit dosage form and may be prepared by any of the methods well known in the art of pharmacy. A unit dosage form may comprise 2.0 mg to 2.0 g, for example 5.0 mg to 300.0 mg of active ingredient. Such methods include the step of bringing into association the active ingredient, ie. the compound of the invention, with the carrier and/or excipients which constitute one or more accessory ingredients. In general the formulations are prepared by uniformly and intimately bringing into association the active ingredient with liquid carriers or finely divided solid carriers and/or excipients and/or two or all of these, and then, if necessary, shaping the product.

Formulations in accordance with the present invention suitable for oral administration may be presented as discrete units such as capsules, cachets or tablets, each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder (e.g. povidone, gelatin, hydroxypropylmethyl cellulose), lubricant, inert diluent, preservative, disintegrant (e.g. sodium starch glycollate, PVP, cross-linked povidone, cross-linked sodium carboxymethyl cellulose), surface-active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein using, for example, hydroxypropylmethylcellulose in varying proportions to provide desired release profile.

Formulations suitable for topical administration in the mouth include lozenges comprising the active ingredient in a flavoured basis, usually sucrose and acacia or tragacanth; pastilles comprising the active ingredient in an inert basis such as gelatin and glycerin, or sucrose and acacia; and mouth-washes comprising the active ingredient in a suitable liquid carrier.

Formulations suitable for parenteral administration include aqueous and non-aqueous sterile injection solutions which may contain anti-oxidants, buffers, bacteriostats and solutes which may render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilised) condition requiring only the addition of the sterile liquid carrier, for example water for injections, immediately prior to use. Extemporaneous injection solutions and suspensions may be prepared from sterile powders, granules and tablets of the kind previously described.

Preferred unit dosage formulations are those containing a daily dose or unit, daily sub-dose or an appropriate fraction thereof, of an active ingredient.

It should be understood that in addition to the ingredients particularly mentioned above the formulations of this invention may include other agents conventional in the art having regard to the type of formulation in question, for example those suitable for oral administration may include flavouring agents.

At least some of the compounds are useful as anticancer, antiviral and/or antiparasitic drugs and at least some of the anticancer compounds can be used against most malignancies.

Particular tumours suitable for treatment in accordance with the invention include leukaemias, and cancers of the uterine cervix, head, neck, brain gliomas, breast, colon, lung, prostate, skin, mouth, nose, oesophagus, stomach, liver, pancreas and metastatic forms of any of these.

Particular viral infections suitable for treatment in accordance with the invention include those caused by the viruses herpes simplex virus I (HSV I); herpes simplex virus II (HSV II); varicella-zoster virus/Ellen (VZV Ellen); bovine papilloma virus (BPV); and human immunodeficiency virus (HIV).

Particular protozoal infections suitable for treatment in accordance with the invention include trichomoniasis; malaria (especially that caused by Plasmodium falciparum); trypanosomiasis (caused by Trypanosoma brucei and T. cruzi); and leishinaniasis. It will be appreciated by those skilled in the art that the novel profile of activity of the present compounds will make it likely that some at least will be useful as antibacterial agents.

A further aspect of the present invention provides a method of treating a human or animal body in need of therapy for a disorder selected from the group consisting of cancer, viral infection or parasitic infection comprising administering to said human or animal body an effective therapeutic dose of a compound or preparation of the invention.

The invention will now be described by way of illustration only by way of the following Examples, Tables and Figures.

### FIGURES:

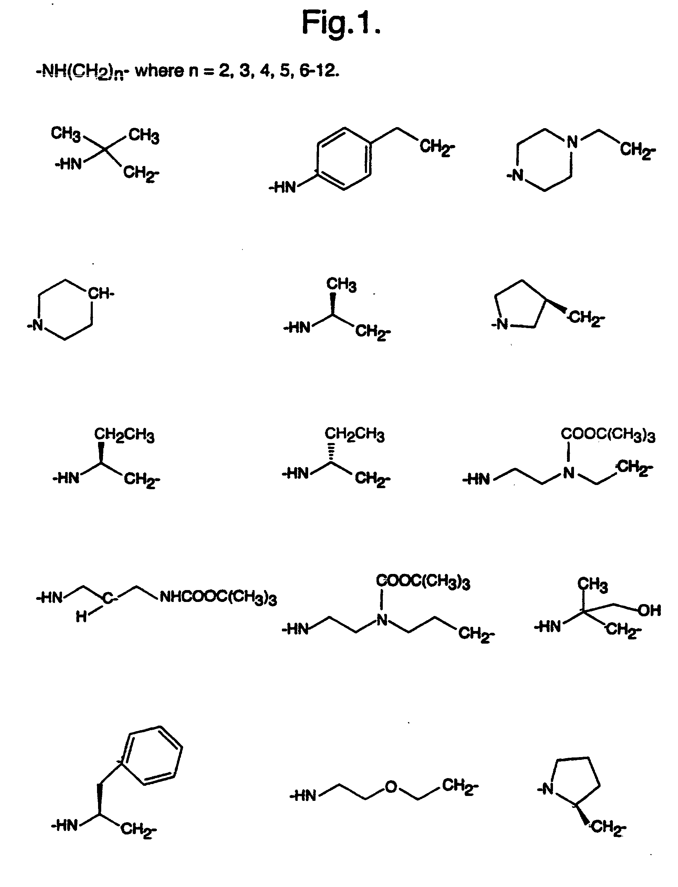

FIGURE 1: shows examples of the moiety -amino-R⁷- where the link to the anthracene ring is through the nitrogen atom '-N' and the link to -O- is through the other free valency shown '-'.

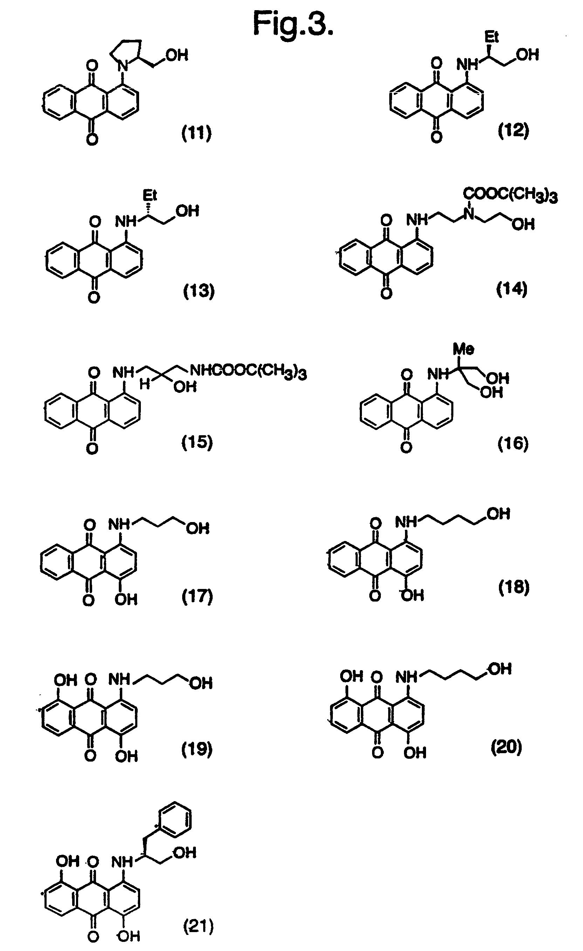

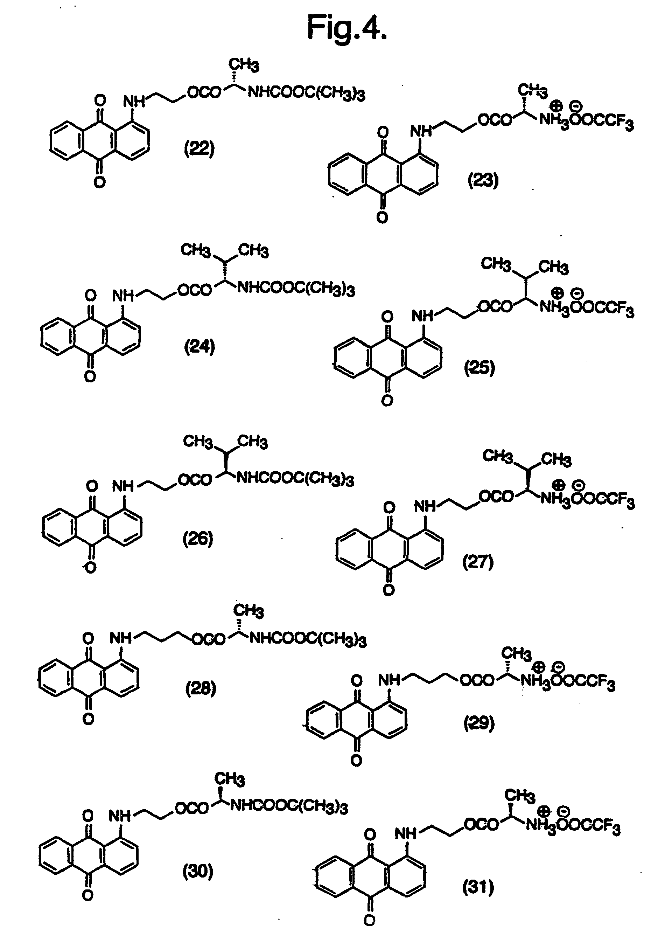

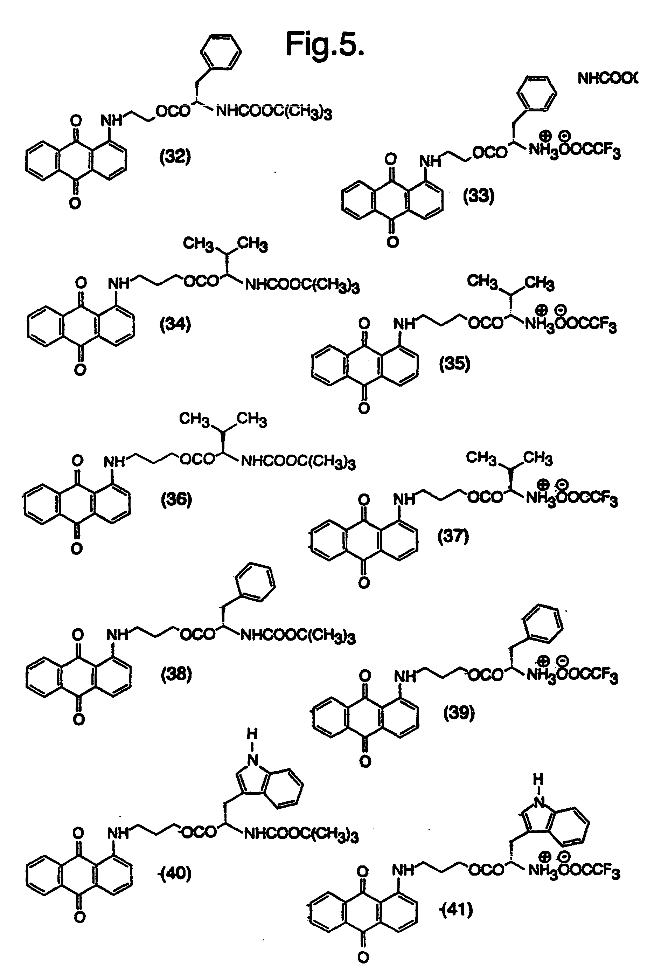

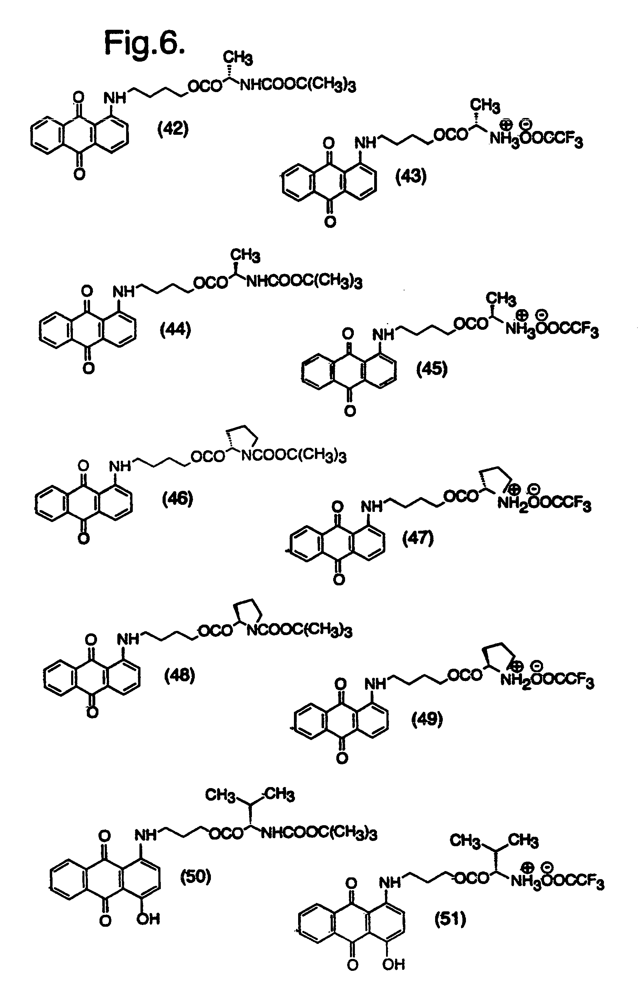

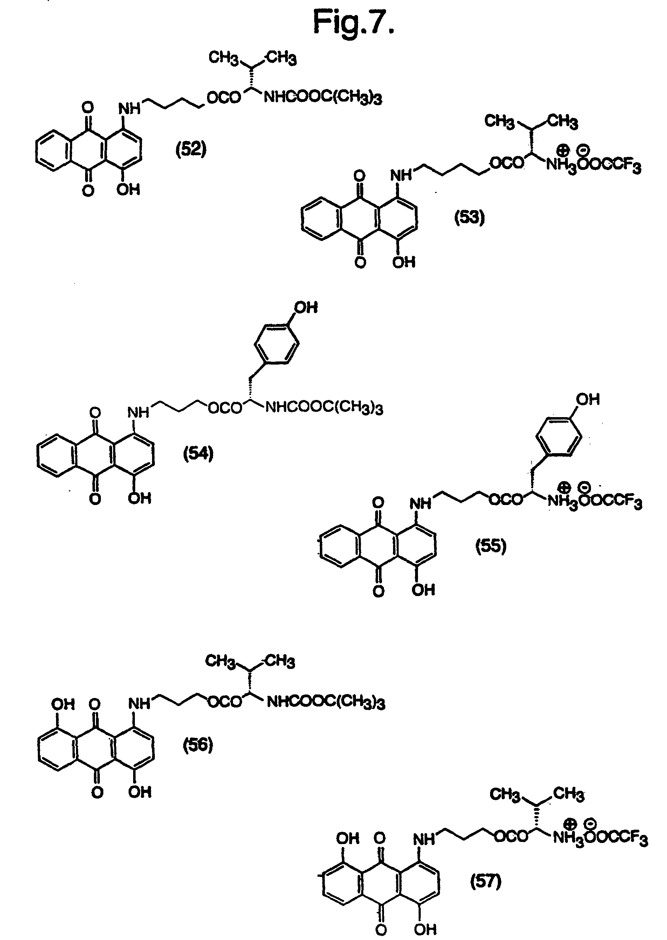

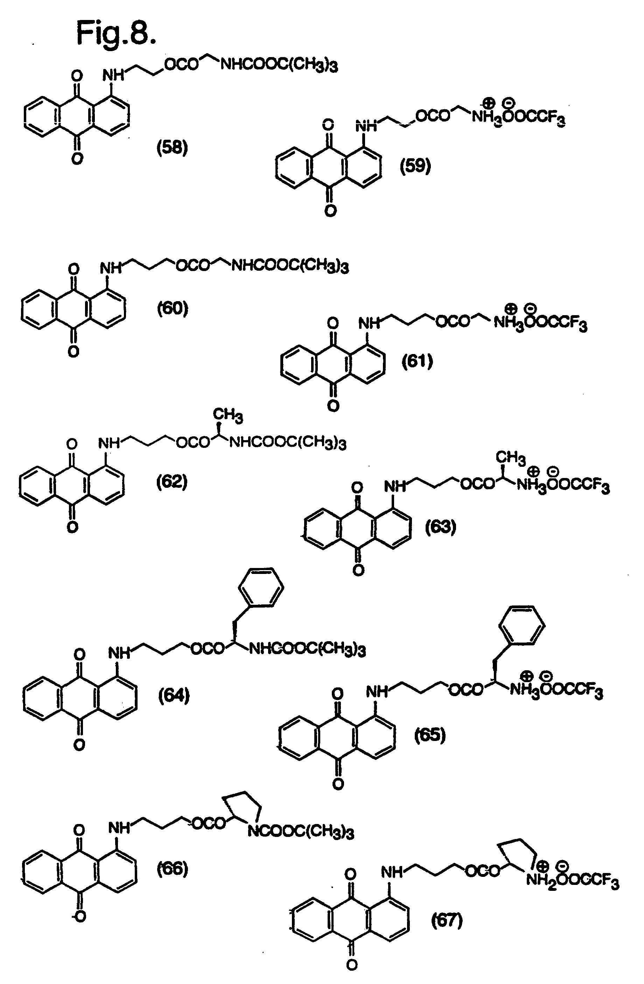

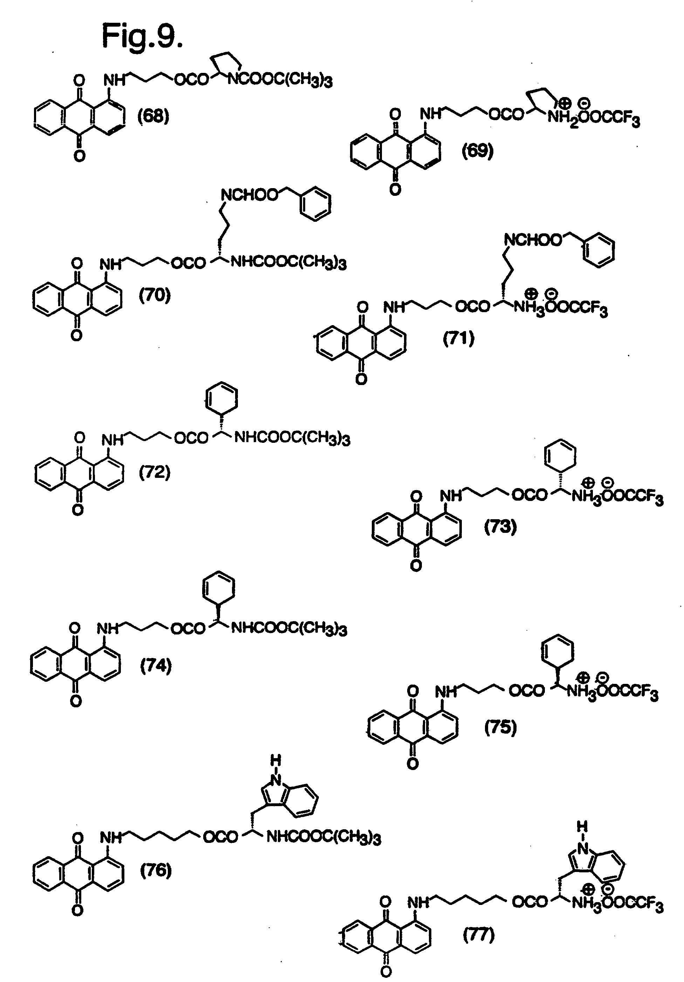

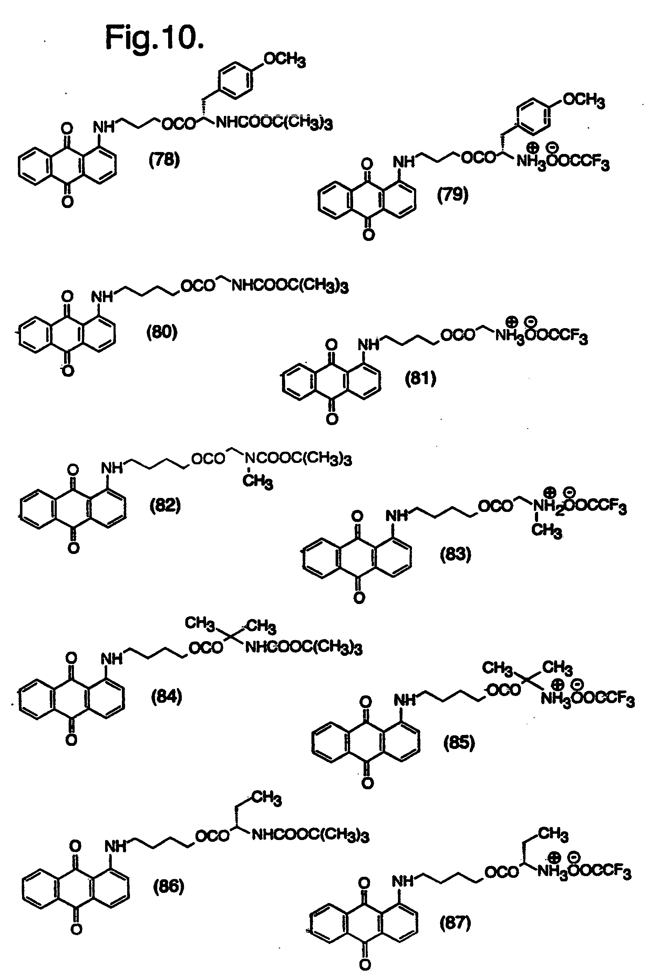

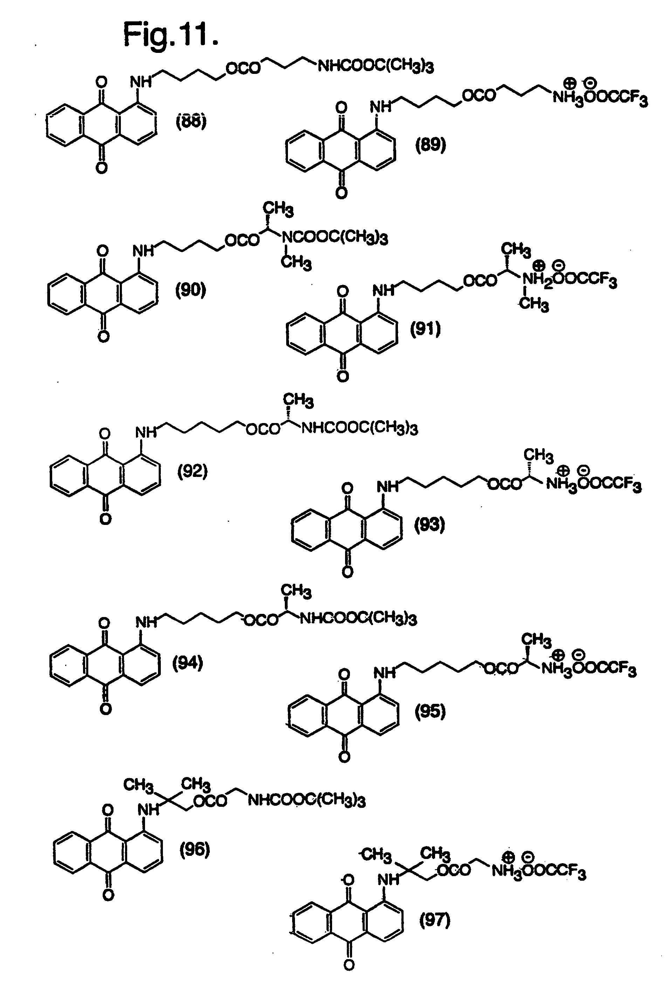

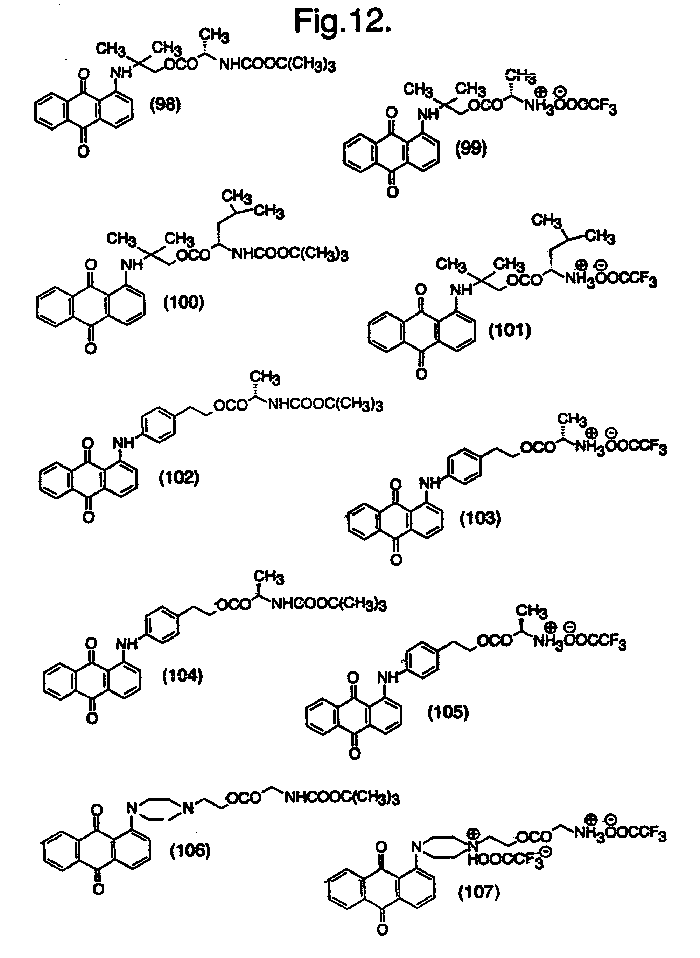

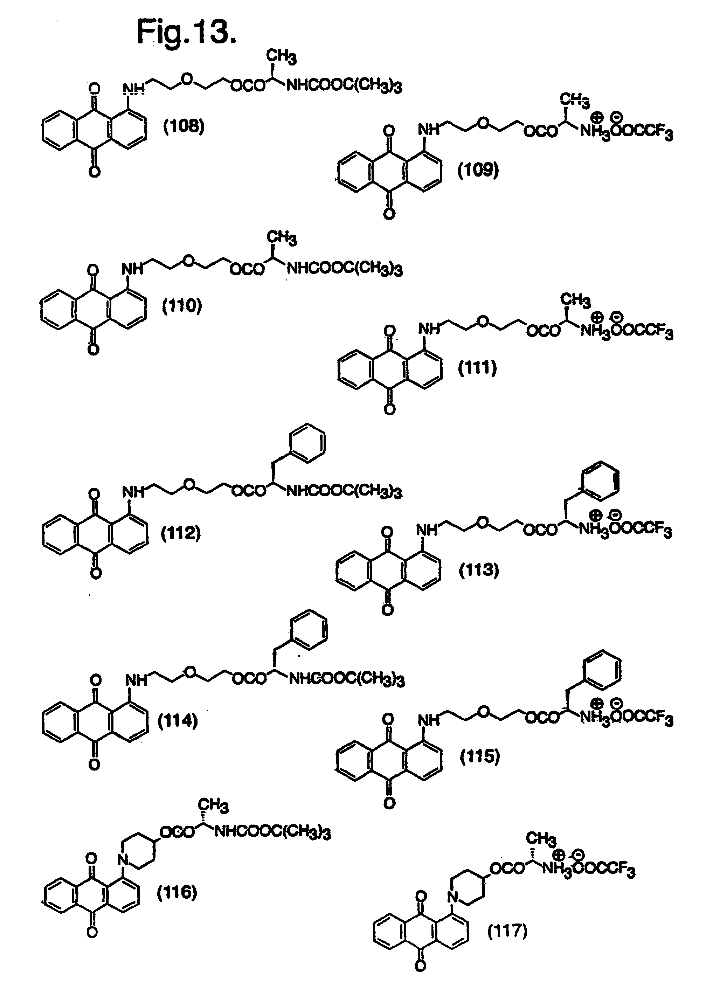

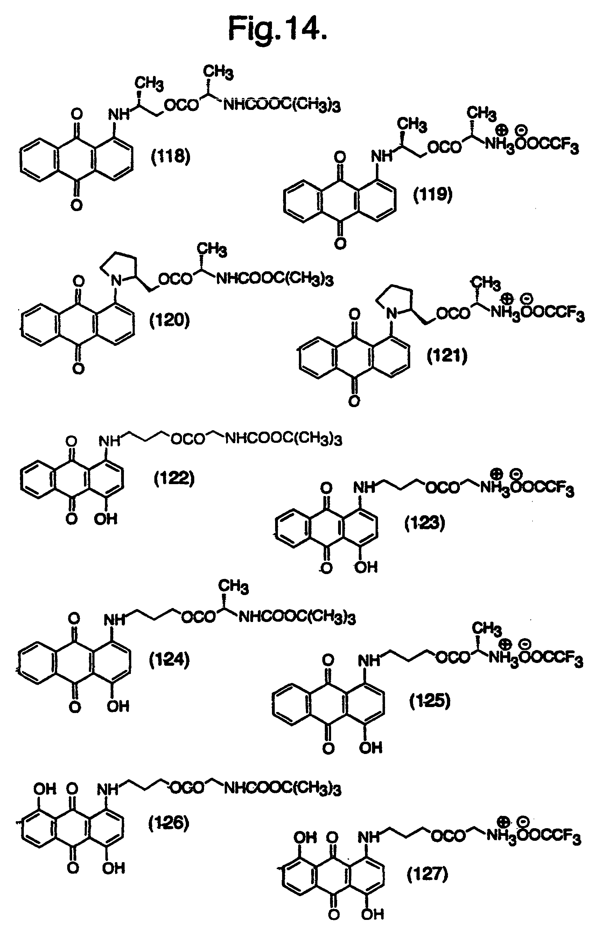

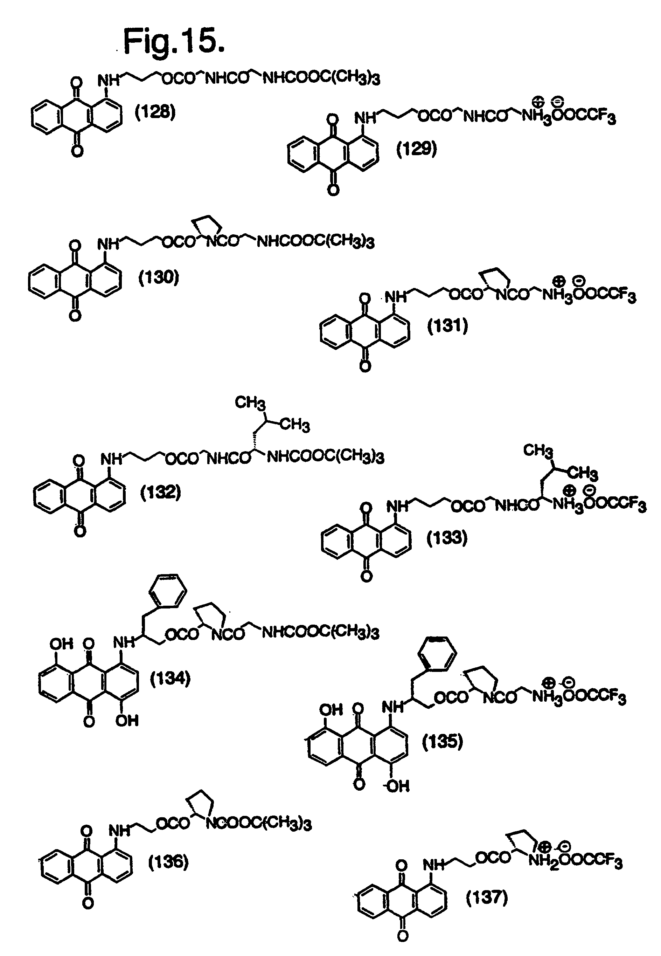

FIGURES 2 to 15 show the structures of the intermediates and product compounds of the invention numbered in accordance with the numbered examples below.
FIGURE 16 shows a graph of relative MAC15A tumour volume v time after a single *in vivo* dose of test compound at the maximum tolerated dose. NU:UB 24, 40 and 44 are preferred amide compounds of US 5,733,880 while NU:UB73 is compound 43 of the present examples.
FIGURE 17 shows a graph of relative MAC15A tumour volume v time after a single *in vivo* dose of test compound at the maximum tolerated dose. NU:UB 43 is a preferred amide compound of US 5,733,880 while NU:UB76 is compound 45 of the present examples

The following specific Examples illustrate preferred, non-limiting compounds and processes of the invention. Further examples falling within the scope of the claims will occur to those in the light of these. Methods 1 to 3 are provided to illustrate the general method for obtaining variously substituted anthracene ring compounds with amino-R⁷-O- spacers attached thereto at the 1-position. Examples 1 to 21 describe preparation of specific anthraquinone intermediates having different hydroxy substitution patterns on the anthraquinone ring with a variety of spacers at the 1-position. Examples 22 to 140 describe the preparation of anthraquinones of the invention possessing the spacer group -amino R⁷-O- linked by the amino -N- to the 1-position and by-O- to amino acids and peptides. These methods can also be used with isosteres such as peptoids and despeptides.

NMR data is provided for certain key compounds but where it is not given this is for reason of brevity; NMRs obtained for examples are consistent with the structures described and shown in the Tables and Figures.

### Method 1 Preparation of 1-[(hydroxy-R⁷)-amino]anthracene-9,10-dione

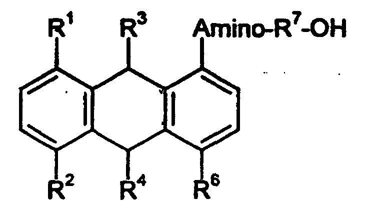

The method described below was used to prepare a number of compounds of the above formula in which the nature and length of the chain of atoms separating the -amino- group from the -O- atom was varied.

1-chloroanthraquinone (10mM) was suspended in DMSO (5 cm³) and an ω-aminoalkanol or an ω-cyclicaminoalcohol (350mM) was then added. The mixture was heated at reflux for 2 hours, cooled and then added to a large excess of water (500 cm³). The red precipitated solid of 1-[(hydroxy-R⁷-amino]anthracene-9,10-dione was filtered off and recrystallised from ethanol.

### Method 2: Preparation of 4-hydroxy-1-[(hydroxy-R⁷)-amino] anthracene-9,10-dione

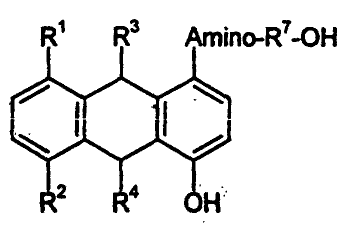

The method described below was used to prepare a number of compounds of the above formula in which the length of the chain of atoms separating the amino group from the -O- atom was varied.

1,4-dihydroxyanthraquinone (10 mM) and an ω-aminoalkanol or cyclicamino R⁷ -alcohol (120 mM) were suspended in ethanol (50 cm³) and THF (50 cm³) and heated over a water bath at 95°C for 1.75 hours. The solution was cooled and immediately applied to a silica gel chromatography column using toluene/ethyl acetate as the eluting solvent to give 4-hydroxy-1-[(hydroxy-R⁷)amino]anthracene-9,10-dione as a purple solid after recrystallisation from ethanol.

### Method 3 Preparation of 4,8-dihydroxy-1-[(hydroxy-R⁷)amino] anthracene-9,10-dione

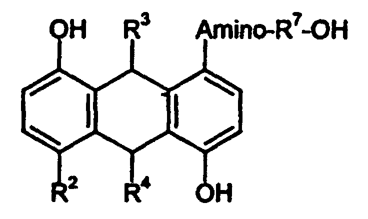

The method described below was used to prepare a number of compounds of the above formula in which the length of the chain separating the amino group from the -O- group was varied.

Leuco-1,4,5-trihydroxyanthraquinone (4mM) was dissolved in dichloromethane (250 cm³) at room temperature, under nitrogen and an ω-aminoalkanol (4mM) was then added. The reaction was stirred for 24 hours at room temperature. At the end of this period, triethylamine (0.5 cm³) was added and the solution was aerated for 2 hours whereupon the colour changed from green to purple. The solvent was evaporated to a low volume and was applied to a silica-gel chromatography column and eluted with dichloromethane with an increasing gradient of toluene-ethyl acetate (4:1). Fractions containing the major products were combined, filtered and evaporated to dryness and the residue was recrystallised from ethanol to give the title compound.

Examples 1 to 21 below describe production of anthraquinone intermediates which have a group -amino-R⁷-O-attached at the position R⁵ in Formula II
Example 1: 1-(2-hydroxyethylamino)anthracene-9,10-dione: Method 1. Prepared using ethanolamine and 1-chloroanthraquinone .The FAB(+) mass spectrum had m/z: 268 (M^{⊕}+1). M, 267.
Example 2: 1-(3-hydroxpropylamino)anthracene-9,10-dione: Method 1. Prepared by the reaction of 3-amino-1-propanol with 1-chloroanthraquinone. C₁₇H₁₅NO₃ requires C,71.9, H,4.9, N,5.0%. Found; C,71.5, H, 4.9, N, 4.9%.:
Example 3: 1-(4-hydroxybutylamino)anthracene-9,10-dione: Method 1. Prepared using 4-amino-1-butanol and 1-chloroanthraquinone. The FAB(+) mass spectrum had m/z: 296 (M^{⊕}+1). M, 295.
Example 4: 1-(5-hydroxypentylamino)anthracene-9,10-dione: Method 1. Prepared using 5-amino-1-pentanol and 1-chloroanthraquinone. The FAB(+) mass spectrum had m/z: 310 (M^{⊕}+1). M, 309.
Example 5:1-[2-(2-hydroxyethoxy)ethylamino]anthracene-9,10-dione: Method 1. Prepared using 2-(2-aminoethoxy)ethanol and 1-chloroanthraquinone. The FAB(+) mass spectrum had m/z: 312 (M^{⊕}+1). M, 311.
Example 6: 1-[(2-hydroxy-tert-butyl)amino]anthracene-9,10-dione: Method 1. Prepared using 2-amino-2-methyl-1-propanol and 1-chloroanthraquinone. The FAB(+) mass spectrum had m/z: 296 (M^{⊕}+1). M, 295.
Example 7:1-{[4-(2-hydroxyethyl)phenyl]amino}anthracene-9,10-dione: Method 1. Prepared using 2-(4-aminophenyl)ethanol and 1-chloroanthraquinone. The low resolution CI(+) mass spectrum m/z: 344 (8%)(M^{⊕}+1). 330 (8%) 72(100%). M, 343.
Example 8: 1-[4-(2-hydroxyethyl)piperazinyl]anthracene-9,10-dione: Method 1. Prepared using 1-(2-hydroxyethyl)piperazine and 1-chloroanthraquinone. The FAB(+) mass spectrum had m/z: 337 (M^{⊕}+1). M, 336.
Example 9: 1-(4-hydroxypiperidyl)anthracene-9,10-dione: Method 1. Prepared using 4-hydroxypiperidine and 1-chloroanthraquinone. The FAB(+) mass spectrum had m/z: 308 (M^{⊕}+1). M, 307.
Example 10: 1-[((1S)-2-hydroxy-isopropyl)amino]anthracene-9,10-dione: Medtod 1. Prepared using (S)-2-amino-1-propanol and 1-chloroanthraquinone. The crude product was purified by column chromatography eluting with chloroform : methanol (20:1). mp 202 °C. The FAB(+) mass spectrum had m/z: 282 (100%)(M^{⊕}+1). M, 281.
Example 11:1-[(2S)-2-(hydroxymethyl)pyrrolidinyl]anthracene-9,10-dione: Method 1. Example (11) was prepared using L-prolinol, 1-chloroanthraquinone and pyridine (1 eq). mp 134 °C. The FAB(+) mass spectrum had m/z: 330 (15%)(M+Na), 308 (100%)(M^{⊕}+1). M, 307.
Example 12: 1-[((1S)-1-ethyl-2-hydroxyethyl)amino]anthracene-9,10-dione: Method 1. Prepared using (S)-2-amino-1-butanol and 1-chloroanthraquinone. The FAB(+) mass spectrum had m/z: 296 (M^{⊕}+1). M, 295.
Example 13: 1-[((1R)-1-ethyl-2-hydroxyethyl)amino]anthracene-9,10-dione: Method 1. Example (13) was prepared using (R)-2-amino-1-butanol and 1-chloroanthraquinone. The FAB(+) mass spectrum had m/z: 296 (M^{⊕}+1). M, 295.
Example 14: (tert-butoxy)-N-{2-[(9,10-dioxoanthryl)amino]ethyl}-N-(2-hydroxy ethyl)carboxamide: Method 1. Prepared using 2-(2-aminoethylamino)ethanol and 1-chloroanthraquinone. The crude product was dissolved in methanol and reacted with ditertiarybutyldicarbonate (3eq) to give the N-^{t}Boc protected compound. The ¹H nmr spectrum (CDCl₃)(200MHz) had δ: 1.5 (9H, s, Boc); 2.95 (1H, br.s, OH); 3.45 (2H, t, CH₂NH); 3.55 (4H, m, CH₂N(Boc)CH₂); 3.75 (2H, t, CH₂ OH); 7.15 (1H, D, H-2); 7.55 (2H, M, H-3, H-4); 7.7 (2H, m, H-6,H-7); 8.25 (2H, m, H-5,H-8); 9.8 (1H, t, NH). The FAB(+) mass spectrum had m/z: 411 (100%)(M^{⊕}+1). 433 (26%) 311 (37%)
Example 15: (tert-butoxy)-N-{3-[(9,10-dioxoanthryl)amino]-2-hydroxy propyl} carboxamide: Method 1. Prepared using 1,3-diamino-2-propanol and 1-chloroanthraquinone. The crude product was dissolved in methanol and reacted with ditertiarybutyldicarbonate (3eq) to give the N-^{t}Boc protected compound, mp 110 °C. Low resolution CI(+) mass spectrum m/z: 397 (50%)(M^{⊕}+1). 195 (100%). M, 396.
Example 16: 1-{[-2-hydroxy-1-(hydroxymethyl)-isopropyl]amino]anthracene-9,10-dione: Method 1. Prepared using 2-amino-2-methylpropane-1,3-diol and 1-chloroanthraquinone. The FAB(+) mass spectrum had m/z: 312 (M^{⊕}+1). M, 311.
Example 17: 4-hydroxy-1-(3-hydroxypropylamino)anthracene-9,10-dione: Method II. Example (17) was prepared using 1,4-dihydroxyanthraquinone and 3-amino-1-propanol. The ¹H nmr spectrum (C₂D₆SO)(200MHz) had δ: 1.5 (2H, quintet, CH₂CH₂CH₂); 3.45 (2H, q, CH₂NH); 3.55 (2H,q,CH₂OH); 4.70 (1H, t, OH); 7.30 (1H, d, H-2); 7.45 (1H, d, H-3); 7.85 (2H, m, H-6, H-7); 8.2 (2H, m, H-5, H-8); 10.7 (1H, t, NH); 13.65 (1H, s, 4-OH).
Example 18: 4-hydroxy-1-(4-hydroxybutylamino)anthracene-9,10-dione: Method II. Example (18) was prepared using 1,4-dihydroxyanthraquinone and 4-amino-1-butanol. The FAB(+) mass spectrum had m/z: 312 (M^{⊕}+1). M, 311.
Example 19: 4,8-dihydroxy-1-[(3-hydroxypropyl)amino]anthracene-9,10-dione: Method III. Prepared using 1-amino-3-propanol and leuco-1,4,5-trihydroxyanthraquinone. C₁₇H₁₅NO₅ requites C,65.2, H,4.8, N,4.5%. Found; C,65.1, H, 4.7, N, 4.5%.
Example 20: 4,8-dihydroxy-1-[(4-hydroxybutyl)amino]anthracene-9,10-dione: Method III. Prepared using 1-amino-4-butanol and leuco-1,4,5-trihydroxyanthraquinone. mp 168 °C
Example 21: 4,8-dihydroxy-1-{[(S-2-hydroxy-1-benzylethyl]amino}anthracene-9,10-dione Method III. Prepared using (S)-2-amino-3-phenyl-1-propanol (L-phenylalaninol) and leuco-1,4,5-trihydroxyanthraquinone. mp 140 °C. The ¹H nmr spectrum (CDCl₃)(200MHz) had δ: 2.35 (1H, t, OH): 3.05 (2H, m, CH₂-phe); 3.85 (2H, m, CH₂OH); 4.05 (1H, m, NHCH); 7.00-7.35 (8H, unresolved, C₆H₅, H-2, H-3 and H-7); 7.55 (1H, d, H-6); 7.75 (1H, d, H-5); 10.20 (1H, d, AQNH); 13.20 (1H, s, 4-OH); 13.80 (1H, s, 8-OH). FAB(+) mass spectrum m/z: 390 (100%)(M^{⊕}+1). M, 389.

### PREPARATION OF ANTHRAQUINONE-HYDROXYALKYLAMINO-SPACER-LINKED PEPTIDE CONJUGATES

Example 22: 1-[2-(N-tertiarybutoxycarbonyl-L-alanyloxy)ethylamino] anthracene-9,10-dione. Dicyclohexylcarbodiimide (DCC) (3.3mM) and 4-dimethylaminopyridine (DMAP) (0.15mM) in dichloromethane (35 cm³) were added to a cooled stirred solution of 1-(2-hydroxyethylamino)anthracene-9,10-dione (3mM) and N-tertiarybutoxycarbonyl-L-alanine (3.3mM) in dichloromethane (35 cm³). Stirring was continued for 12h as the mixture was allowed to reach room temperature. The precipitated dicyclohexylurea (DCU) was filtered off and the solution partitioned between chloroform and water (1:1, 100 cm³), washed three times with water (50 cm³), dried (MgSO₄), filtered and evaporated to dryness. The solid was dissolved in toluene and applied to a silica-gel chromatography column and eluted with an increasing gradient of toluene-ethyl acetate (4:1). Recrystallisation from ethanol afforded the title compound (22) as orange/red crystals. Yield 80%.
   The ¹H nmr spectrum (CDCl₃)(200MHz) had δ: 1.40 (12H, s, ^{t}Boc and CH₃-ala); 3.68 (2H, q, Ar-NH-CH₂); 4.15 (1H, q, α-CH); 4.40(2H, t, CH₂-O); 5.08 (1H, br. d, NH-Boc); 7.10 (1H, dd, H-2); 7.78 (3H, m, H-3, H-6 and H-7): 8.25 (3H, m, H-4, H-5 and H-8); 9.90 (1H, t, Ar-NH). C₂₄H₂₆N₂O₆ requires C,65.7, H,6.0, N,6.4%. Found; C,65.4, H, 6.4, N, 6.0%.:The FAB(+) mass spectrum had: m/z 439 (M^{⊕}+1). M, 438.
Example 23: 1-[2-(L-alanyloxy)ethylamino]anthracene-9,10-dione trifluoroacetate. The ^{t}Boc protected example 22 (0.50g) was dissolved in trifluoroacetic acid (10cm³) at room temperature. After 0.5h the solvent was evaporated and the solid reevaporated from ethanol (3×10 cm³) before dissolving in a minimum volume of ethanol (3 cm³). Addition of ether (100 cm³) gave a red precipitate of 1-[2-(alanyloxy)ethylamino]anthracene-9,10-dione trifluoroacetate salt which was filtered off, washed with cold ether and dried (0.41g)(79%). The ¹H nmr (d₆-DMSO)(200MHz) spectrum had 8: 1.40 (3H, d, CH₃-ala); 3.72 (2H, t, Ar-NH-CH₂); 4.15 (1H, q, α-CH); 4.40 (2H, m, CH₂O); 7.40 (1H, dd, H-2); 7.8 (2H, m, H-3 and H-4); 8.15 (2H, m, H-6 and H-7); 8.4 (2H, m, H-5 and H-8); 9.75 (1H, t, Ar-NH). C₂₁H₁₉F₃N₂O₆ requires C,55.8, H,4.2, N,6.2%. Found; C,55.5, H,4.1, N,6.0%. The FAB(+) mass spectrum had: m/z 339 (R-NH₃^{⊕}).
   The following examples [(24)-(49)] were prepared by an equivalent procedure to that described for example (22), in the N-protected form, except that the synthesis began with the appropriate N-tertiarybutoxycarbonyl-protected amino acid in place of N-^{t}Boc-L-ala, and the appropriate anthraquinone spacer compound from examples 1-16, followed by N-deprotection of the first formed ester conjugate using trifluoroacetic acid by analogous procedures to the preparation of Example 23.
   Example 24: 1-[2-(N-tertiarybutoxycarbonyl-L-valyloxy)ethylamino]anthracene-9,10-dione. Prepared from anthraquinone-spacer compound (1) and N-tertiarybutoxycarbonyl-L-valine by an equivalent procedure to that described for example 22.
   Example 25: 1-[2-(L-valyloxy)ethylamino]anthracene-9,10-dione trifluoroacetate. Prepared by deprotection of example 24 by an equivalent procedure to that described for example 23. The ¹H nmr spectrum (d₆DMSO)(200MHz) had δ: 0.95 (6H, d, 2xCH₃); 2.2 (1H, m, β-CH); 3.7 (2H, q, ArNHCH₂); 4.0 (1H, d, α-CH); 4.5 (2H, m, CH₂OCO); 7.35 (1H, d, H-2); 7.45 (1H, d, H-4); 7.65 (1H, t, H-3); 7.85-8.0(2H, m, H-6, H-7); 8.10-8.25 (2H, m, H-5, H-8); 8.5 (3H, br.s, ⁺NH₃); 9.80 (1H, t, AQNH). The FAB(+) mass spectrum had: m/z 367 (R-NH₃^{⊕}).
Example 26: 1-[2-(N-tertiarybutoxycarbonyl-D-valyloxy)ethylamino]anthracene-9,10-dione. Prepared from anthraquinone-spacer compound (1) and N-tertiarybutoxycarbonyl-O-valine by an equivalent procedure to that described for example 22.
Example 27: 1-[2-(D-valyloxy)ethylamino]anthracene-9,10-dione trifluoroacetate. Prepared by deprotection of example 26 by an equivalent procedure to that described for example 23. The ¹H nmr spectrum (d₆DMSO)(200MHz) had δ: 0.95 (6H, d, 2xCH₃); 2.2 (1H, m, β-CH); 3.7 (2H, q, ArNHCH₂); 4.0 (1H, d, α-CH); 4.5 (2H, m, CH₂OCO); 7.35 (1H, d, H-2); 7.45 (1H, d, H-4); 7.65 (1H, t, H-3); 7.85-8.0(2H, m, H-6, H-7); 8.10-8.25 (2H, m, H-5, H-8); 8.5 (3H, br.s, ⁺NH₃); 9.80 (1H, t, AQNH). The FAB(+) mass spectrum had: m/z 367 (R-NH₃^{⊕}).
Example 28: 1-[3-(N-tertiarybutoxycarbonyl-L-alanyloxy)propylamino] anthracene-9,10-dione. Prepared from anthraquinone-spacer compound (2) and N-tertiarybutoxycarbonyl-L-alanine by an equivalent procedure to that described for example 22. mp 60 °C C₂₅H₂₈N₂O₆ requires C 66.4, H 6.2, N 6.2%. Found C 66.1, H 6.3, N6.1%. The FAB(+) mass spectrum had m/z: 453 (7%)(M^{⊕}+1), 397 (12%), 57 (100%). M, 452.
Example 29: 1-[3-(L-alanyloxy)propylamino]anthracene-9,10-dione trifluoroacetate. Prepared by deprotection of example 28 by an equivalent procedure to that described for example 23. mp 126 °C. The ¹H nmr spectrum (d₆-DMSO)(200MHz) had δ: 1.20 (3H, d, CH₃-ala); 1.80 (2H, quintet, -CH₂-CH₂-CH₂-); 3.30 (2H, q, ArNH-CH₂); 3.85 (1H, q, α-CH); 4.05 (2H, t, CH₂-OCO); 7.05 (1H, dd, H-2); 7.22 (1H, dd, H-4); 7.45 (1H, t, H-3); 7.65 (2H, m, H-6 and H-7); 7.90 (2H, m, H-5 and H-8); 8.35 (3H, Br.s, NH₃⁺); 9.50 (1H, t, ArNH). The FAB(+) mass spectrum had m/z: 353 (100%)(RNH₃^{⊕}), 282 (28%), 44 (79%).
Example 30: 1-[2-(N-tertiarybutoxycarbonyl-D-alanyloxy)ethylamino] anthracene-9,10-dione. Prepared from anthraquinone-spacer compound (1) and N-tertiarybutoxycarbonyl-D-alanine by an equivalent procedure to that described for example 22. The FAB(+) mass spectrum had: m/z 439 (M^{⊕}+1). M, 438.
Example 31: 1-[2-(D-alanyloxy)ethylamino] anthracene-9,10-dione trifluoroacetate. Prepared by deprotection of example 30 by an equivalent procedure to that described for example 23. The ¹H nmr (d₆-DMSO)(200MHz) spectrum had δ: 1.40 (3H, d, CH₃-ala); 3.72 (2H, t, Ar-NH-CH₂); 4.15 (1H, q, α-CH); 4.40 (2H, m, CH₂O); 7.40 (1H, dd, H-2); 7.8 (2H, m, H-3 and H-4); 8.15 (2H, m, H-6 and H-7); 8.4 (2H, m, H-5 and H-8); 9.75 (1H, t, Ar-NH). The FAB(+) mass spectrum had: m/z 339 (R-NH₃^{⊕}).
Example 32: 1-[2-(N-tertiarybutoxycarbonyl-L-phenylalanyloxy)ethylamino] anthracene-9,10-dione .Prepared from anthraquinone-spacer compound (1) and N-tertiarybutoxycarbonyl-L-phe by an equivalent procedure to that described for example 22.
Example 33: 1-[2-(L-phenylalanyloxy)ethylamino]anthracene-9,10-dione trifluoroacetate. Prepared by deprotection of example 32 by an equivalent procedure to that described for example 23. The ¹H nmr spectrum (d₆-DMSO)(200MHz) had δ: 3.1 (2H, d, CH₂Ph); 3.7 (2H, m, ArNHCH₂); 4.35 (3H, m, unresolved, α-CH, CH₂OCO); 7.2 (5H, s, Ph); 7.3 (1H, d, H-3); 7.45 (H, d, H-4); 7.7 (1H, t, H-3); 7.90 (2H, m, H-6, H-7); 8.15 (2H, m, H-5,H-8); 8.6 (3H, br.s, NH₃⁺); 9.85 (1H, t, AQNH). The FAB(+) mass spectrum had: m/z 415 (R-NH₃^{⊕}).
Example 34: 1-[3-(N-tertiarybutoxycarbonyl-L-valyloxy)propylamino] anthracene-9,10-dione. Prepared from anthraquinone-spacer compound (2) and N-tertiarybutoxycarbonyl-L-val by an equivalent procedure to that described for example 22.
Example 35: 1-[3-(L-valyloxy)propylamino]anthracene-9,10-dione trifluoroacetate. Prepared by deprotection of example 34 by an equivalent procedure to that described for example 23. The ¹H nmr spectrum (d₆-DMSO)(200MHz) had δ: 0.95 (6H, m, CH₃x2); 1.95 (2H, quintet, NHCH₂CH₂CH₂); 2.10 (1H, m, β-CH); 3.50 (2H, q, ArNHCH₂); 3.95 (1H, d, α-CH); 4.35 (2H, t, CH₂OCO); 7.25 (1H, dd, H-2); 7.45 (1H, dd, H-4); 7.60 (1H, t, H-3); 7.90 (2H, m, H-6, H-7); 8.15 (2H, m, H-5, H-8); 8.50 (3H, br.s, NH₃⁺); 9.70 (1H, t, AQNH). The FAB(+) mass spectrum had: m/z 381 (R-NH₃^{⊕}).
Example 36: 1-[3-(N-tertiarybutoxycarbonyl-D-valyloxy)propylamino] anthracene-9,10-dione Prepared from anthraquinone-spacer compound (2) and N-tertiarybutoxycarbonyl-D-val by an equivalent procedure to that described for example 22.
Example 37: 1-[3-(D-valyloxy)propylamino]anthracene-9,10-dione trifluoroacetate. Prepared by deprotection of example 36 by an equivalent procedure to that described for example 23. The ¹H nmr speetrum (d₆-DMSO)(200MHz) had δ: 0.95 (6H, m, CH₃x2); 1.95 (2H, quintet, NHCH₂CH₂CH₂); 2.10 (1H, m, β-CH); 3.50 (2H, q, ArNHCH₂); 3.95 (1H, d, α-CH); 4.35 (2H, t, CH₂OCO); 7.25 (1H, dd, H-2); 7.45 (1H, dd, H-4); 7.60 (1H, t, H-3); 7.90 (2H, m, H-6, H-7); 8.15 (2H, m, H-5, H-8); 8.50 (3H, br.s, NH₃⁺); 9.70 (1H, t, AQNH). The FAB(+) mass spectrum had: m/z 381 (R-NH₃^{⊕}).
Example 38: 1-[3-(N-tertiarybutoxycarbonyl-L-phenylalanyloxy)propylamino]-anthracene-9,10-dione.
   Prepared from anthraquinone-spacer compound (2) and N-tertiary-butoxycarbonyl-L-phe by an equivalent procedure to that described for example 22. The FAB(+) mass spectrum had: m/z 529 (100%)(M^{⊕}+1).
Example 39: 1-[3-(L-phenylalanyloxy)propylamino]anthracene-9,10-dione trifluoroacetate. Prepared by deprotection of example 38 by an equivalent procedure to that described for example 23. The ¹H nmr spectrum (d₆DMSO)(200MHz) had δ: 1.95 (2H, t, CH₂CH₂CH₂); 3.15 (2H, dd, CH₂Ph); 3.4( 2H, q, NHCH₂); 4.2 (2H, t, CH₂OCO); 4.35 (1H, t, α-CH); 7.15-7.3 (6H, m, unresolved, Ph, H-2); 7.45 (1H, d, H-4); 7.68 (1H, t, H-3); 7.90 (2H, m, H-6, H-7); 8.2(2H, m, H-5, H-8); 8.6 (3H, br.s, NH₃⁺); 9.65 (1H, t, AQNH). The FAB(+) mass spectrum had: m/z 429 (R-NH₃^{⊕}).
Example 40: 1-[3-(N-tertiarybutoxycarbonyl-L-tryptophyloxy)propylamino] anthracene-9,10-dione .Prepared from anthraquinone-spacer compound (2) and N-tertiarybutoxycarbonyl-L-trp by an equivalent procedure to that described for example 22.
Example 41: 1-[3-(L-tryptophyloxy)propylamino]anthracene-9,10-dione trifluoroacetate. Prepared by deprotection of example 40 by an equivalent procedure to that described for example 23. The ¹H nmr spectrum (d₆DMSO)(200MHz) had δ: 1.95 (2H, quintet, NHCH₂); 3.4 (4H, m, unresolved, ArNHCH₂, β-CH₂), 4.2 (2H, q, CH₂OCO); 4.35 (1H, t, α-CH); 6.9-7.2 (4H, m, unresolved, H-2, H-4(AQ), H-5, H-6(indole)); 7.3 (1H, s, H-2(indole)); 7.4-7.6 (2H, m, unresolved, H-4, H-7(indole)); 7.65 (1H, t, H-3); 7.85 (2H, m, H-6, H-7); 8.15 (2H, m, H-5, H-8); 8.65 (3H, br.s, NH₃⁺); 9.7 (1H, t, AQNH); 11.1 (1H, s, NH(indole)). The FAB(+) mass spectrum had: m/z 468 (R-NH₃^{⊕}).
Example 42: 1-[4-(N-tertiarybutoxycarbonyl-L-alanyloxy)butylamino] anthracene-9,10-dione. .Prepared from anthraquinone-spacer compound (3) and. N-tertiarybutoxycarbonyl-L-ala by an equivalent procedure to that described for example 22. mp 102 °C. The FAB(+) mass spectrum had m/z: 467 (M^{⊕}+1). M, 466.
Example 43: 1-[4-(L-alanyloxy)butylamino]anthracene-9,10-dione trifluoroacetate. Prepared by deprotection of example 42 by an equivalent procedure to that described for example 23. The ¹H nmr spectrum (d₆-DMSO)(200MHz) had δ: 1.20 (3H, d, CH₃-ala); 1.5-1.7 (4H, m, -CH₂-CH₂-CH₂-CH₂-); 3.20 (2H, m, ArNH-CH₂); 4.10 (2H, m, CH₂-OCO); 4.20 (1H, m, α-CH); 7.25 (1H, dd, H-2); 7.40 (1H, dd, H-4); 7.60 (1H, m, H-3); 7.7-7.9 (2H, m, H-6 and H-7); 8.1 (3H, br. s, NH₃^{⊕}); 8.20-8.40 (2H, m, H-5 and H-8). The FAB(+) mass spectrum had: m/z 367 (R-NH₃^{⊕}).
Example 44: 1-[4-(N-tertiarybutoxycarbonyl-D-alanyloxy)butylamino] anthracene-9,10-dione. Prepared from anthraquinone-spacer compound (3) and N-tertiarybutoxycarbonyl-D-ala by an equivalent procedure to that described for example 22. mp 100 °C The FAB(+) mass spectrum had m/z: 467 (M^{⊕}+1). M, 466.
Example 45: 1-[4-(D-alanyloxy)butylamino]anthracene-9,10-dione trifluoroacetate. Prepared by deprotection of example 44 by an equivalent procedure to that described for example 23. The ¹H nmr spectrum (d₆-DMSO)(200MHz) had δ: 1.20 (3H, d, CH₃-ala); 1.5-1.7 (4H, m, -CH₂-CH₂-CH₂-CH₂-); 3.20 (2H, m, ArNH-CH₂); 4.10 (2H, m, CH₂-OCO); 4.20 (1H, m, α-CH); 7.25 (1H, dd, H-2); 7.40 (1H, dd, H-4); 7.60 (1H, m, H-3); 7.7-7.9 (2H, m, H-6 and H-7); 8.1 (3H, br. s, NH₃^{⊕}); 8.20-8.40 (2H, m, H-5 and H-8). The FAB(+) mass spectrum had: m/z 367 (R-NH₃^{⊕}).
Example 46: 1-[4-(N-tertiarybutoxycarbonyl-L-prolyloxy)butylamino] anthracene-9,10-dione. Prepared from anthraquinone-spacer compound (3) and N-tertiarybutoxycarbonyl-L-pro by an equivalent procedure to that described for example 22. The FAB(+) mass spectrum had m/z: 493 (M^{⊕}+1). M, 492.
Example 47: 1-[4-(L-prolyloxy)butylamino]anthracene-9,10-dione trifluoroacetate. Prepared by deprotection of example 46 by an equivalent procedure to that described for example 23. The ¹H nmr spectrum (d₆-DMSO)(200MHz) had δ: 1.5-1.7 (4H, m, -CH₂-CH₂-CH₂-CH₂-); 2.25 (4H, m, β-CH₂, γ-CH₂); 3.20 (2H, m, ArNH-CH₂ and δ-CH₂); 4.10 (2H, m, CH₂-OCO); 4.35 (1H, m, α-CH); 7.15 (1H, dd, H-2); 7.40 (1H, dd, H-4); 7.65 (1H, t, H-3); 7.80-8.0 (2H, m, H-6 and H-7); 8.1 (3H, br. s, NH₃^{⊕}); 8.20-8.40 (2H, m, H-5 and H-8); 9.85 (1H, t, Ar-NH). The FAB(+) mass spectrum had: m/z 393 (R-NH₃^{⊕}).
Example 48: 1-[4-(N-tertiarybutoxycarbonyl-D-prolyloxy)butylamino] anthracene-9,10-dione. Prepared from anthraquinone-spacer compound (3) and N-tertiarybutoxycarbonyl-D-pro by an equivalent procedure to that described for example 22. The FAB(+) mass spectrum had m/z: 493 (M^{⊕}+1). M, 492.
Example 49: 1-[4-(D-prolyloxy)butylamino]anthracene-9,10-dione trifluoroacetate. Prepared by deprotection of example 48 by an equivalent procedure to that described for example 23. The FAB(+) mass spectrum had: m/z 393 (R-NH₃^{⊕}).
   The following examples [(50)-(55)] were prepared by an equivalent procedure to that described for example (22), in the N-protected form, except that the synthesis began with the appropriate N-tertiarybutoxycarbonyl-protected amino acid in place of N-^{t}Boc-L-ala, and the appropriate anthraquinone spacer compound from examples 17-18, followed by N-deprotection of the first formed ester conjugate using trifluoroacetic acid by analogous procedures to the preparation of example 23.
Example 50: 4-hydroxy-1-[3-(N-tertiarybutoxycarbonyl-L-valyloxy)propyl amino]anthracene-9,10-dione. Prepared from anthraquinone-spacer compound (17) and N-tertiary-butoxycarbonyl-L-val by an equivalent procedure to that described for example 22. The FAB(+) mass spectrum had: m/z 497 (M^{⊕}+1).
Example 51: 4-hydroxy-1-[3-(L-valyloxy)propylamino]anthracene-9,10-dione trifluoroacetate. Prepared by deprotection of example 50 by an equivalent procedure to that described for example 23. The FAB(+) mass spectrum had: m/z 397 (R-NH₃^{⊕}). The ¹H nmr spectrum (d₆-DMSO)(200MHz) had δ: 1.0 (6H, t, CH₃x2); 2.05 (2H, m, CH₂CH₂CH₂); 2.2 (1H, m, β-CH); 3.55 (2H, m, CH₂NH); 4.0 (1H, d, α-CH); 4.35 (2H, t, CH₂O); 7.35 (1H, d, H-2); 7.48 (2H, d, H-3); 7.8-7.96 (2H, m, H-6, H-7); 8.18-8.26 (2H, m, H-5, H-8); 8.5 (3H, br.s, NH₃⁺); 10.26 (1H, t, NH); 13.6 (1H, s, OH).
Example 52: 4-hydroxy-1-[4-(N-tertiarybutoxycarbonyl-L-valyloxy)butylamino]anthracene-9,10-dione. Prepared from anthraquinone-spacer compound (18) and N-tertiary-butoxycarbonyl-L-val by an equivalent procedure to that described for example 22. The FAB(+) mass spectrum had: m/z 511 (M^{⊕}+1).
Example 53: 4-hydroxy-1-[4-(L-valyloxy)butylamino]anthracene-9,10-dione trifluoroacetate. Prepared by deprotection of example 52 by an equivalent procedure to that described for example 23. The FAB(+) mass spectrum had: m/z 411 (R-NH₃^{⊕}). The ¹H nmr spectrum (d₆-DMSO)(200MHz) had δ: 0.95 (6H, t, CH₃x2); 1.8 (4H, m,-CH₂CH₂); 3.95 (1H, m, α-CH); 4.3 (2H, m, CH₂); 7.35 (1H, d, H-2); 7.5 (1H, d, H-3); 7.82-7.98 (2H, m, H-6, H-7); 8.20-8.30 (2H, m, H-5, H-8); 8.45 (3H, br.s, NH₃⁺); 10.28 (1H, t, NH); 13.65 (1H, s, OH).
Example 54: 4-hydroxy-1-[3(N-tertiarybutoxycarbonyl-L-tyrosyloxy)propylamino]anthracene-9,10-dione. Example 54 was prepared from anthraquinone-spacer compound (17) and N-tertiary-butoxycarbonyl-L-tyr by an equivalent procedure to that described for example 22.
Example 55: 4-hydroxy-1-[3-(L-tyrosyloxy)propylamino]anthracene-9,10-dione trifluoroacetate. Prepared by deprotection of example 54 by an equivalent procedure to that described for example 23. The ¹H nmr spectrum (d₆DMSO)(200MHz) had δ: 1.95 (2H, quintet, NHCH₂CH₂); 2.75 (2H, m, β-CH₂-tyr); 3.30(2H, m, ArNHCH₂); 3.55 (1H, t, α-CH); 4.15 (2H, t, CH₂-OCO); 6.6 (2H, d, H-3', H-5',tyr); 6.95 (2h, d, H-2', H-6', tyr); 7.35 (1H, d, H-2); 7.45 (1H, d, H-3); 7.95 (2H, m, H-6, H-7); 8.2 (2H, m, H-5, H-8); 8.6 (3H, br.s, NH₃); 9.20 (1H, s, 1-OH); 10.25 (1H, t, AQNH); 13.5 (1H, s, 4-OH). The FAB(+) mass spectrum had: m/z 461 (R-NH₃^{⊕}).
   The following examples [(56)-(57)] were prepared by an equivalent procedure to that described for example (22), in the N-protected form, except that the synthesis began with the appropriate N-tertiarybutoxycarbonyl-protected amino acid in place of N-^{t}Boc-L-ala, and the appropriate anthraquinone spacer compound example 19, followed by N-deprotection of the first formed ester conjugate using trifluoroacetic acid by analogous procedures to the preparation of example 23.
Example 56: 4,8-dihydroxy-1-[3-(N-tertiarybutoxycarbonyl-L-valyloxy)propylamino]anthracene-9,10-dione Prepared from anthraquinone-spacer compound (19) and N-tertiary-butoxycarbonyl-L-val by an equivalent procedure to that described for example 22.
Example 57: 4,8-dihydroxy-1-[3-(L-valyloxy)propylamino]anthracene-9,10-dione trifluoroacetate. Prepared by deprotection of example 56 by an equivalent procedure to that described for example 23. The ¹H nmr spectrum (d₆-DMSO)(200MHz) had δ: 0.95 (6H, overlapping doublets, 2xCH₃); 2.0 (2H, quintet, CH₂CH₂CH₂); 2.2 (1H, m, β-CH); 3.55 (2H, q, ArNHCH₂); 3.95 (1H, d, α-CH); 4.30 (2H, t, CH₂OCO); 7.25 (2H, m, unresolved H-2, H-7); 7.45 (1H, d, H-3); 7.65 (2H, m, unresolved, H-5, H-6); 8.5 (3H, br.s, NH₃⁺); 9.85 (1H, t, AQNH); 13.2 (1H, s, 4-OH); 13.85 (1H, s, 8-OH). The FAB(+) mass spectrum had: m/z 413 (R-NH₃^{⊕}).
   The following examples [(58)-(121)] were prepared by an equivalent procedure to that described for example (22), in the N-protected form, except that the synthesis began with the appropriate N-tertiarybutoxycarbonyl-protected amino acid in place of N-^{t}Boc-L-ala, and the appropriate anthraquinone spacer compound from examples 1-16, followed by N-deprotection of the first formed ester conjugate using trifluoroacetic acid by analogous procedures to the preparation of example 23.
Example 58: 1-[2-(N-tertiarybutoxycarbonylglycyloxy)ethylamino]anthracene-9,10-dione. Prepared from anthraquinone-spacer compound (1) and N-tertiarybutoxycarbonyl-gly by an equivalent procedure to that described for example 22. mp 122 °C. The low resolution CI(+) mass spectrum had m/z: 425 (37%)(M^{⊕}+1), 210 (100%). The accurate mass measurement CI peak match [M+H] (reference compound: perfluorotributylamine) had: Calculated mass m/z 425.1712. Measured mass m/z: 425.1705.
Example 59: 1-[2-(glycyloxy)ethylamino]anthracene-9,10-dione trifluoroacetate. Prepared by deprotection of example 58 by an equivalent procedure to that described for example 23. mp 180 °C. The ¹H nmr spectrum (d₆-DMSO)(200MHz) had δ: 3.70 (2H, q, ArNH-CH₂); 3.80 (2H, s, CH₂-gly); 4.38 (2H, t, CH₂-OCO); 7.30 (1H, dd, H-2); 7.42 (1H, dd, H-4); 7.62 (1H, t, H-3); 7.80 (2H, m, H-6 and H-7); 8.15 (2H, m, H-5 and H-8); 8.35 (3H, Br.s, NH₃⁺); 9.75 (1H, t, ArNH). The electrospray (+)(Cone 50V) mass spectrum had m/z: 325 (75%)(RNH₃^{⊕}), 250 (100%). The electrospray (-)(Cone -20V) mass spectrum had m/z: 113 (100%).
Example 60: 1-[3-(N-tertiarybutoxycarbonylglycyloxy)propylamino]anthracene-9,10-dione. Prepared from anthraquinone-spacer compound (2) and N-tertiarybutoxycarbonyl-gly by an equivalent procedure to that described for example 22. mp 84 °C C₂₄H₂₆N₂O₆ requires C 65.7, H 6.0, N,6.4%. Found C 65.7, H 6.0, N 6.4%. The FAB(+) mass spectrum had m/z: 439 (4%)(M^{⊕}+1), 57 (100%). M, 438.
Example 61: 1-[3-(glycyloxy)propylamino]anthracene-9,10-dione trifluoroacetate. Example 61 was prepared by deprotection of example 60 by an equivalent procedure to that described for example 23. mp 198 °C. The ¹H nmr spectrum (d₆-DMSO)(200MHz) had δ: 2.05 (2H, quintet, -CH₂-CH₂-CH₂-); 3.50 (2H, q, ArNH-CH₂); 3.88 (2H, s, CH₂-gly); 4.32 (2H, t, CH₂-OCO); 7.25 (1H, dd, H-2); 7.40 (1H, dd, H-4); 7.65 (1H, t, H-3); 7.85 (2H, m, H-6 and H-7); 8.15 (2H, m, H-5 and H-8); 8.35 (3H, Br.s, NH₃⁺); 9.75 (1H, t, ArNH). C₂₁H₁₉N₂O₆F₃ requires C 55.8, H 4.2, N 6.2%. Found C 56.1, H 4.0, N6.2%. The FAB(+) mass spectrum had m/z: 339 (100%)(RNH₃^{⊕}).
Example 62: 1-[3-(N-tertiarybutoxycarbonyl-D-alanyloxy)propyl amino] anthracene-9,10-dione. Prepared from anthraquinone-spacer compound (2) and N-tertiarybutoxycarbonyl-D-ala by an equivalent procedure to that described for example 22. mp 58 °C. C₂₅H₂₈N₂O₆ requires C 66.4, H 6.2, N 6.2%. Found C 65.8, H 6.1, N6.0%. The FAB(+) mass spectrum had m/z: 453 (10%)(M^{⊕}+1), 57 (100%). M, 452.
Example 63: 1-[3-(D-alanyloxy)propylamino]anthracene-9,10-dione trifluoroacetate. Prepared by deprotection of example 62 by an equivalent procedure to that described for example 23. mp 134 °C. The ¹H nmr spectrum (d₆-DMSO)(200MHz) had δ: 1.40 (3H, d, CH₃-ala); 2.02 (2H, quintet, -CH₂-CH₂-CH₂-); 3.50 (2H, q, ArNH-CH₂); 4.15 (1H, q, α-CH); 4.30 (2H, t, CH₂-OCO); 7.22 (1H, dd, H-2); 7.40 (1H, dd, H-4); 7.60 (1H, t, H-3); 7.80 (2H, m, H-6 and H-7); 8.05 (2H, m, H-5 and H-8); 8.35 (3H, Br.s, NH₃⁺); 9.70 (1H, t, ArNH). C₂₂H₂₁N₂O₆F₃ requires C 56.7, H 4.5, N 6.0%. Found C 56.3, H 4.4, N6.0%. The FAB(+) mass spectrum had m/z: 353 (82%)(RNH₃^{⊕}), 282 (28%), 236 (36%), 44 (100%).
Example 64: 1-[3-(N-tertiarybutoxycarbonyl-D-phenylalanyloxy)propylamino]-anthracene-9,10-dione.Prepared from anthraquinone-spacer compound (2) and N-tertiary-butoxycarbonyl-D-phe by an equivalent procedure to that described for example 22. mp 168°C. The FAB(+) mass spectrum had m/z: 529 (100%)(M^{⊕}+1), 551 (33%), 473(54%).
Example 65: 1-[3-(D-phenylalanyloxy)propylamino]anthracene-9,10-dione trifluoroacetate. Prepared by deprotection of example 64 by an equivalent procedure to that described for example 23. mp 102°C. The ¹H nmr spectrum (d₆DMSO)(200MHz) had δ: 1.95 (2H, t, CH₂CH₂CH₂); 3.15 (2H, dd, CH₂Ph); 3.4( 2H, q, NHCH₂); 4.2 (2H, t, CH₂OCO); 4.35 (1H, t, α-CH); 7.15-7.3 (6H, m, unresolved, C₆H₅, H-2); 7.45 (1H, d, H-4); 7.68 (1H, t, H-3); 7.90 (2H, m, H-6, H-7); 8.2(2H, m, H-5, H-8); 8.6 (3H, br.s, NH₃⁺); 9.65 (1H, t, AQNH). The electrospray (+)(Cone 50V) mass spectrum had m/z: 429 (100%)(RNH₃^{⊕}), 451 (10%). The electrospray (-)(Cone -20V) mass spectrum had m/z: 113 (14%), 69 (100%).
Example 66: 1-[3-(N-tertiarybutoxycarbonyl-L-prolyloxy)propyl amino]anthracene-9,10-dione. Prepared from anthraquinone-spacer compound (2) and N-tertiarybutoxycarbonyl-L-pro by an equivalent procedure to that described for example 22. mp 121 °C. C₂₇H₃₀N₂O₆ requires C 67.8, H 6.3, N 5.9%. Found C 67.2, H 6.5, N6.0%. The FAB(+) mass spectrum had m/z: 479 (86%)(M^{⊕}+1), 422 (37%), 379 (47%), 197 (100%). M, 478.
Example 67: 1-[3-(L-prolyloxy)propylamino]anthracene-9,10-dione trifluoroacetate. Prepared by deprotection of example 66 by an equivalent procedure to that described for example 23. Mp 66°C. The ¹H nmr spectrum (d₆-DMSO)(200MHz) had δ: 1.95 (5H, unresolved, -CH₂-CH₂-CH₂- and γ-CH₂-pro, β-CH-pro); 2.28 (1H, m, β-CH'-pro); 3.25 (2H, m, δ-CH₂-pro), 3.40 (2H, q, ArNH-CH₂); 4.40 (3H, unresolved, α-CH, CH₂-OCO); 7.25 (1H, dd, H-2); 7.40 (1H, dd, H-4); 7.60 (1H, t, H-3); 7.80 (2H, m, H-6, H-7); 8.10 (2H, m, H-5, H-8); 9.65 (1H, t, ArNH). The electrospray (+)(Cone 50V) mass spectrum had m/z: 379 (100%)(RNH₃^{⊕}), 264 (50%). The electrospray (-)(Cone - 20V) mass spectrum had m/z: 113 (100%).
Example 68: 1-[3-(N-tertiarybutoxycarbonyl-D-prolyloxy)propylamino]anthracene-9,10-dione. Prepared from anthraquinone-spacer compound (2) and N-tertiarybutoxycarbonyl-D-pro by an equivalent procedure to that described for example 22. mp 120 °C. C₂₇H₃₀N₂O₆ requires C 67.8, H 6.3, N 5.9%. Found C 67.2, H 6.5, N6.0%. The FAB(+) mass spectrum had m/z: 479 M^{⊕}+1), 422 379, 197. M, 478. C₂₇H₃₀N₂O₆ requires C 67.8, H 6.3, N 5.9%. Found C 67.5, H 6.5, N 6.0%.
Example 69: 1-[3-(D-prolyloxy)propylamino]anthracene-9,10-dione trifluoroacetate. Prepared by deprotection of example 68 by an equivalent procedure to that described for example 23. Mp 66 °C. The ¹H nmr spectrum (d₆-DMSO)(200MHz) had δ: 1.95 (5H, unresolved, -CH₂-CH₂-CH₂-, β-CH-pro and γ-CH₂-pro); 2.28 (1H, m, β-CH'-pro); 3.25 (2H, m, δ-CH₂-pro); 3.40 (2H, q, ArNH-CH₂); 4.34 (2H, t, CH₂-OCO); 4.45 (1H, t, α-CH); 7.25 (1H, dd, H-2); 7.40 (1H, dd, H-4); 7.65 (1H, t, H-3); 7.80 (2H, m, H-6 and H-7); 8.10 (2H, m, H-5 and H-8); 9.68 (1H, t, ArNH). The electrospray (+)(Cone 50V) mass spectrum had m/z: 379 (100%)(RNH₃^{⊕}), 264 (50%). The electrospray (-)(Cone -20V) mass spectrum had m/z: 113 (100%).
Example 70: 1-[3-(N-α-tertiarybutoxycarbonyl-N-δ-benzyloxycarbonyl-L-ornithyloxy)propylamino]anthracene-9,10-dione. Prepared from anthraquinone-spacer compound (2) and N-α-tertiary-butoxycarbonyl-N-δ-benzyloxycarbonyl-L-orn by an equivalent procedure to that described for example 22. mp 130 °C. C₃₅H₃₉N₃O₈ requires C 66.8, H 6.2, N 6.7%. Found C 66.8, H 6.2, N 6.6%. The FAB(+) mass spectrum had m/z: 630 (51%)(M^{⊕}+1), 530 (8%), 107 (100%). M, 629.
Example 71: 1-[3-(N-δ-benzyloxycarbonyl-L-ornithyloxy)propylamino] anthracene-9,10-dione trifluoroacetate. Prepared by deprotection of example 70 by an equivalent procedure to that described for example 23. mp 80 °C. The ¹H nmr spectrum (d₆-DMSO)(200MHz) had δ: 1.40-1.70 (4H, unresolved, β-CH₂ and γ-CH₂-orn); 2.00 (2H, quintet, -CH₂-CH₂-CH₂-); 2.98 (2H, q, δ-CH₂-orn); 3.45 (2H, m, ArNH-CH₂) 3.60 (1H, m, and α-CH); 4.20 (2H, t, CH₂-OCO); 5.00 (2H, s, CH₂-Z); 7.25 (6H, unresolved, H-2 and C₆H₅); 7.40 (1H, dd, H-4); 7.60 (1H, t, H-3); 7.82 (2H, m, H-6 and H-7); 8.12 (2H, m, H-5 and H-8); 9.70 (1H, t, ArNH). The electrospray (+)(Cone 20V) mass spectrum had m/z: 530 (100%)(RNH₃^{⊕}), 97 (45%). The electrospray (-)(Cone -20V) mass spectrum had m/z: 113 (100%).
Example 72: 1-[3-(N-tertiarybutoxycarbonyl-L-phenylglycyloxy)propylamino]anthracene-9,10-dione. Prepared from anthraquinone-spacer compound (2) and N-tertiary-butoxycarbonyl-L-phg by an equivalent procedure to that described for example 22. The FAB(+) mass spectrum had m/z: 515 (100%)(M^{⊕}+1), 537 (26%), 415 (17%). M, 514.
Example 73: 1-[3-(L-phenylglycyloxy)propylamino]anthracene-9,10-dione trifluoroacetate. Prepared by deprotection of example 72 by an equivalent procedure to that described for example 23. The ¹H nmr spectrum (d₆-DMSO)(200MHz) had δ: 1.95 (2H, quintet, CH₂); 3.40 (2H, m, CH₂NH); 4.30 (2H, t, CH₂OCO); 5.4 (1H, s, α-CH); 7.10 (1H, dd, H-2); 7.3-7.7 (7H, m, unresolved, Ph, H-3, H-4); 7.85 (2H, m, H-6,H-7); 8.15 (2H, m, H-5, H-8); 8.95 (3H, br.s., NH₃); 9.65 (1H, t, ArNH). The electrospray (+)(Cone 20V) mass spectrum had m/z: 415 (100%)(RNH₃^{⊕}), 437 (9%). The electrospray (-)(Cone -20V) mass spectrum had m/z: 113 (8%), 69 (100%).
Example 74: 1-[3-(N-tertiarybutoxycarbonyl-D-phenylglycyloxy)propylamino]anthracene-9,10-dione. Prepared from anthraquinone-spacer compound (2) and N-tertiary-butoxycarbonyl-D-phg by an equivalent procedure to that described for example 22. The FAB(+) mass spectrum had m/z: 515 (46%)(M^{⊕}+1), 538 (26%), 262.2 (100%). M, 514.
Example 75: 1-[3-(D-phenylglycyloxy)propylamino]anthracene-9,10-dione trifluoroacetate. Prepared by deprotection of example 74 by an equivalent procedure to that described for example 23. The ¹H nmr spectrum (d₆-DMSO)(200MHz) had δ: 1.95 (2H, quintet, CH₂); 3.40 (2H, m, CH₂NH); 4.30 (2H, t, CH₂O); 5.4 (1H, s, α-CH); 7.10 (1H, dd, H-2); 7.3-7.7 (7H, m, unresolved, Ph, H-3, H-4); 7.85 (2H, m, H-6,H-7); 8.15 (2H, m, H-5, H-8); 8.95 (3H, br.s., NH₃); 9.65 (1H, t, ArNH). The electrospray (+)(Cone 20V) mass spectrum had m/z: 415 (100%)(RNH₃^{⊕}), 100 (10%). The electrospray (-)(Cone -20V) mass spectrum had m/z: 113 (100%), 69 (20%).
Example 76: 1-[5-(N-tertiarybutoxycarbonyl-L-tryptophyloxy)pentoxyamino]anthracene-9,10-dione Prepared from anthraquinone-spacer compound (4) and N-tertiary-butoxycarbonyl-L-trp by an equivalent procedure to that described for example 22. The FAB(+) mass spectrum had m/z: 596 (62%)(M^{⊕}+1), 618 (21%), 100 (100%). M, 595.
Example 77: 1-[5-(L-tryptophyloxy)pentoxyamino]anthracene-9,10-dione trifluoroacetate. Prepared by deprotection of example 76 by an equivalent procedure to that described for example 23. The ¹H nmr spectrum (d₆-DMSO)(200MHz) had δ: 1.3 (2H, m, CH₂CH₂CH₂ CH₂CH₂); 1.55 (4H, m, CH₂ CH₂ CH₂CH₂CH₂); 3.70 (2H, dd, CH₂); 3.5 (2H, m, CH₂); 4.05 (2H, t, CH₂O); 4.3 (1H, t, α-H); 6.95-7.10 (2H, m, unresolved, H-2(AQ), H-2(indole); 7.3-7.5 (3H, m, unresolved, H-4(AQ), H-4, H-7(indole)); 7.65 (1H, t, H-3); 7.85 (2H, m, H-6, H-7); 8.15 (2H, m, H-5, H-8); 8.45 (3H, br.s, NH₃⁺); 9.7 (1H, t, NH); 11.1 (1H, s, NH(indole)). The electrospray (+)(Cone 20V) mass spectrum had m/z: 496 (100%)(RNH₃^{⊕}), 219 (28%). The electrospray (-)(Cone -20V) mass spectrum had m/z: 113 (100%), 69 (17%).
Example 78: 1-[3-(N-tertiarybutoxycarbonyl-O-methyl-L-tyrosyloxy)propyl amino]anthracene-9,10-dione Prepared from anthraquinone-spacer compound (2) and N-tertiary-butoxycarbonyl-O-methyl-L-tyr by an equivalent procedure to that described for example 22. The FAB(+) mass spectrum had m/z: 559 (62%)(M^{⊕}+1), 581 (46%), 459 (46%). M, 558.
Example 79: 1-[3-(O-methyl-L-tyrosyloxy)propylamino]anthracene-9,10-dione trifluoroacetate. Prepared by deprotection of example 78 by an equivalent procedure to that described for example 23. The ¹H nmr spectrum (d₆-DMSO)(200MHz) had δ: 1.95 (2H, quintet, CH₂); 3.1 (2H, m, CH₂Ph); 3.4 (2H, q, CH₂NH); 3.7 (3H, s, OMe); 4.2-4.45( (3H, m, unresolved, α-H, CH₂OCO); 6.85 (2H, d, H-2',6' o-CH×2); 7.2 (2H, dd, H-2); 7.25 (2H, d, H-3',4' m-CH×2); 7.45 (1H, d, H-4); 7.7 (1H, t, H-3); 7.9 (2H, m, H-6, H-7); 8.2 (2H, m, H-5, H-8); 8.5 (3H, br.s, NH₃); 9.7 (1H, t, NH). The electrospray (+)(Cone 20V) mass spectrum had m/z: 459 (100%)(RNH₃^{⊕}), 210 (10%). The electrospray (-)(Cone -20V) mass spectrum had m/z: 113 (100%), 69 (12%).
Example 80: 1-[4-(N-tertiarybutoxycarbonylglycyloxy)butylamino]anthracene-9,10-dione. Prepared from anthraquinone-spacer compound (3) and N-tertiarybutoxycarbonyl-gly by an equivalent procedure to that described for example 22. mp 70 °C. The low resolution CI(+) mass spectrum had m/z: 453 (17%)(M^{⊕}+1), 70 (100%). M, 452.
Example 81: 1-[4-(glycyloxy)butylamino]anthracene-9,10-dione trifluoroacetate. Prepared by deprotection of example 80 by an equivalent procedure to that described for example 23. Mp 154 °C. The ¹H nmr spectrum (d₆-DMSO)(200MHz) had δ: 1.78 (4H, m, -CH₂-CH₂-CH₂-CH₂-); 3.40 (2H, q, ArNH-CH₂); 3.80 (2H, s, CH₂-gly); 4.22 (2H, t, CH₂-OCO); 7.22 (1H, dd, H-2); 7.42 (1H, dd, H-4); 7.62 (1H, t, H-3); 7.82 (2H, m, H-6 and H-7); 8.12 (2H, m, H-5 and H-8); 9.65 (1H, t, ArNH). C₂₂H₂₁N₂O₆F₃ requires C 56.7, H 4.5, N 6.0%. Found C 56.3, H 4.2, N 6.0%.
Example 82: 1-[4-(N-tertiarybutoxycarbonylsarcosyloxy)butylamino]anthracene-9,10-dione. Prepared from anthraquinone-spacer compound (3) and N-tertiarybutoxycarbonyl-sar by an equivalent procedure to that described for example 22. mp 90 °C. The FAB(+) mass spectrum had m/z: 467 (63%)(M^{⊕}+1), 411 (56%), 278 (100%).
Example 83: 1-[4-(sarcosyloxy)butylamino]anthracene-9,10-dione trifluoroacetate. Prepared by deprotection of example 82 by an equivalent procedure to that described for example 23. mp 132 °C. The ¹H nmr spectrum (d₆-DMSO)(200MHz) had δ:1.75 (4H, m, CH₂CH₂CH₂CH₂); 2.55 (3H, s, NCH₃); 3.45 (2H, q, ArNHCH₂) 3.95 (2H, s, CH₂-sar); 4.30 (2H, t, CH₂OCO); 7.25 (1H, dd, H-2); 7.45 (1H, dd,H-4); 7.65 (1H, t, H-3); 7.95 (2H, m, H-6,H-7); 8.15 (2H, m, H-5, H-8); 9.0 (3H, br.s, NH₃^{⊕}); 9.7 (1H, t, ArNH). The electrospray (+)(Cone 20V) mass spectrum had m/z: 367 (100%)(RNH₃^{⊕}). The electrospray (-)(Cone -50V) mass spectrum had m/z: 113 (12%), 69 (100%).
Example 84: 1-[4-(N-α-tertiarybutoxycarbonyl-α-methylalanyloxy)butylamino]anthracene-9,10-dione. Prepared from anthraquinone-spacer compound (3) and N-tertiary-butoxycarbonyl-α-Me-ala by an equivalent procedure to that described for. example 22. mp 112 °C. The FAB(+) mass spectrum had m/z: 503 (26%)(M^{⊕}+Na), 481 (98%)(M^{⊕}+1),, 278 (100%).
Example 85: 1-[4-(methylalanyloxy)butylamino]anthracene-9,10-dione trifluoroacetate. Prepared by deprotection of example 84 by an equivalent procedure to that described for example 23. The ¹H nmr spectrum (d₆-DMSO)(200MHz) had δ: 1.45 (3H, d, α-CH₃); 1.8 (4H, m, CH₂CH₂CH₂CH₂); 2.58 (3H, s, NCH₃); 3.45 (2H, ArNHCH₂); 4.10 (1H, q, α-CH); 4.70 (2H, t, CH₂OCO); 7.3 (1H, dd, H-2); 7.45 (1H, dd, H-4); 7.7 (1H, t, H-3); 7.85 (2H, m, H-6, H-7); 8.15 (2H, m, H-5, H-8); 9.05 (2H, br.s, NH₂^{⊕}); 9.7 (1H, t, ArNH). The electrospray (+)(Cone 20V) mass spectrum had m/z: 381 (RNH₃^{⊕})(50%); 278(100%). The electrospray (-)(Cone -50V) mass spectrum had m/z: 113 (100%),(CF₃COO⁻).
Example 86: (S)-1-{4-[2-(N-tertiarybutoxycarbonylamino)butanoyloxy] butylamino}-anthracene-9,10-dione. Prepared from anthraquinone-spacer compound (3) and (S)-N-tertiary-butoxycarbonyl-α- aminobutyric acid. mp 58 °C. The FAB(+) mass spectrum had m/z: 481 (66%)(M^{⊕}+1), 425 (78%), 278 (100%).
Example 87: (S)-1-[4-(2-aminobutanoyloxy)butylamino]-anthracene-9,10-dione trifluoroacetate. Prepared by deprotection of example 86 by an equivalent procedure to that described for example 23. mp 118 °C. The electrospray (+)(Cone 8V) mass spectrum had m/z: 381 (100%)(RNH₃^{⊕}). The electrospray (-)(Cone -50V) mass spectrum had m/z: 113 (100%).
Example 88: 1-{4-[4-(N-tertiarybutoxycarbonyl)butanoyloxy] butyl amino} anthracene-9,10-dione Prepared from anthraquinone-spacer compound (3) and 4-N-tertiary-butoxycarbonylbutanoic acid. mp 100 °C. The FAB(+) mass spectrum had m/z: 481 (100%)(M^{⊕}+1). M, 480.
Example 89: 1-[4-(butanoyloxy)butylamino]anthracene-9,10-dione trifluoroacetate. Prepared by deprotection of example 88 by an equivalent procedure to that described for example 23. Mp 111 °C. The ¹H nmr spectrum (d₆-DMSO)(200MHz) had δ: 1.80 (6H, unresolved, CH₂-CH₂-CH₂-CH₂-OCO-CH₂-CH₂); 2.40 (2H, t, OCO-CH₂); 2.80 (2H, t, CH₂-NH₃^{⊕}) 3.45 (2H, q, ArNH-CH₂); 4.10 (2H, t, CH₂-OCO); 7.30 (1H, dd, H-2); 7.45 (1H, dd, H-4); 7.65 (4H, unresolved, H-3 and NH₃^{⊕}); 7.85 (2H, m, H-6 and H-7); 8.15 (2H, m, H-5 and H-8); 9.70 (1H, t, ArNH).
Example 90: 1-[4-(N-tertiarybutoxycarbonyl-N-methyl-L-alanyloxy)butylamino] anthracene-9,10-dione. Prepared from anthraquinone-spacer compound (3) and N-^{t}Boc-N-methyl-L-alanine. mp 62 °C. The FAB(+) mass spectrum had m/z: 481 (96%), 278 (100%)(M^{⊕}+1). M, 480.
Example 91: 1-[4-(N-methyl-L-alanyloxy)butylamino]anthracene-9,10-dione trifluoroacetate. Prepared by deprotection of example 90 by an equivalent procedure to that described for example 23. mp 84 °C. The ¹H nmr spectrum (d₆-DMSO)(200MHz) had δ: 1.45 (6H, s, (CH₃)₂-aib); 1.80 (4H, m, NH-CH₂-CH₂-CH₂); 3.40 (2H, q, ArNH-CH₂); 4.25 (2H, t, CH₂-OCO); 7.30 (1H, dd, H-2); 7.45 (1H, dd, H-4); 7.65 (1H, t, H-3); 7.85 (2H, m, H-6 and H-7); 8.15 (2H, m, H-5 and H-8); 8.50 (3H, br. s, NH₃^{⊕}); 9.70 (1H, t, ArNH). The electrospray (+)(Cone 8V) mass spectrum had m/z: 381 (100%)(RNH₃^{⊕}). The electrospray (-)(Cone -50V) mass spectrum had m/z: 113 (100%).
Example 92: 1-[5-(N-tertiarybutoxycarbonyl-L-alanyloxy)pentylamino] anthracene-9,10-dione Prepared from anthraquinone-spacer compound (4) and N-^{t}Boc-L-alanine. The FAB(+) mass spectrum had m/z: 481 (M^{⊕}+1). M, 480.
Example 93: 1-[5-(L-alanyloxy)pentylamino]anthracene-9,10-dione trifluoroacetate. Prepared by deprotection of example 92 by an equivalent procedure to that described for example 23. The ¹H nmr spectrum (d₆-DMSO)(200MHz) had δ: 1.40 (3H, d, CH,-ala); 1.50 (2H, s, CH₂-CH₂-CH₂-CH₂-CH₂-); 1.70 (4H, m, CH₂-CH₂-CH₂-CH₂-CH₂); 3.30 (2H, q, ArNH-CH₂); 4.05 (2H, t, α-CH-ala); 4.20 (2H, t, CH₂-OCO); 7.13 (1H, dd, H-2); 7.40 (1H, dd, H-4); 7.62 (1H, t, H-3); 7.85 (2H, m, H-6 and H-7); 8.12 (2H, m, H-5 and H-8); 9.60 (1H, t, ArNH). The electrospray (+)(Cone 50V) mass spectrum had m/z: 381 (RNH₃^{⊕}). The electrospray (-)(Cone -20V) mass spectrum had m/z: 113.
Example 94: 1-[5-(N-tertiarybutoxycarbonyl-D-alanyloxy) pentylamino] anthracene-9,10-dione Prepared from anthraquinone-spacer compound (4) and N-^{t}Boc-D-alanine. The FAB(+) mass spectrum bad m/z: 481 (M^{⊕}+1). M, 480.
Example 95: 1-[5-(D-alanyloxy)pentylamino]anthracene-9,10-dione trifluoroacetate. Prepared by deprotection of example 94 by an equivalent procedure to that described for example 23. The ¹H nmr spectrum (d₆-DMSO)(200MHz) had δ: 1.40 (3H, d, CH₃-ala); 1.50 (2H, m, CH₂-CH₂-CH₂-CH₂-CH₂-); 1.70 (4H, m, CH₂-CH₂-CH₂-CH₂-CH₂); 3.30 (2H, q, ArNH-CH₂); 4.05 (2H, t, α-CH-ala); 4.25 (2H, t, CH₂-OCO); 7.15 (1H, dd, H-2); 7.35 (1H, dd, H-4); 7.55 (1H, t, H-3); 7.80 (2H, m, H-6 and H-7); 8.10 (2H, m, H-5 and H-8); 9.60 (1H, t, ArNH). The electrospray (+)(Cone 50V) mass spectrum had m/z: 381 (RNH₃^{⊕}). The electrospray (-)(Cone -20V) mass spectrum had m/z: 113.
Example 96: 2-[(9,10-dioxoanthryl)amino]-2-methylpropyl-2-[(tert-butoxy) carbonyl-amino] acetate. Prepared from anthraquinone-spacer compound (6) and N-^{t}Boc-glycine. mp 114°C C₂₅H₂₈N₂O₆ requires C 66.4, H 6.2, N 6.2%. Found C 66.5, H 6.2, N 6.1%. The low resolution CI(+) mass spectrum had m/z: 453 (17%)(M^{⊕}+1), 70 (100%). M, 452.
Example 97: 2-[(9,10-dioxoanthryl)amino]-2-methylpropyl 2-aminoacetate trifluoroacetate. Prepared by deprotection of example 96 by an equivalent procedure to that described for example 23. mp 130°C. The ¹H nmr spectrum (d₆-DMSO)(200MHz) had δ: 1.50 (6H, s, C(CH₃)₂-CH₂-OCO); 3.40 (2H, s, CH₂-gly); 4.40 (2H, s, CH₂-OCO); 7.55 (3H, unresolved, H-2, H-3 and H-4); 7.85 (2H, m, H-6 and H-7); 8.15 (2H, m, H-5 and H-8); 10.15 (1H, s, ArNH). C₂₂H₂₁N₂O₆F₃ requires C 56.7, H 4.5, N 6.0%. Found C 56.3, H 4.6, N 5.9%. The electrospray (+)(Cone 8V) mass spectrum had m/z: 353 (100%)(RNH₃^{⊕}), 97 (85%). The electrospray (-)(Cone -20V) mass spectrum had m/z: 113 (30%), 91 (100%).
Example 98: 2-[(9,10-dioxoanthryl)amino]-2-methylpropyl (2S)-2-[(tert-butoxy)-carbonylamino]propanoate Prepared from anthraquinone-spacer compound (6) and N-^{t}Boc-L-alanine. mp 120 °C. C₂₆H₃₀N₂O₆ requires C 66.9, H 6.5, N 6.0%. Found C 66.9, H 6.5, N 6.0%. The low resolution CI(+) mass spectrum had m/z: 467 (90%)(M^{⊕}+1), 61 (100%). M, 466.
Example 99: 2-[(9,10-dioxoanthryl)amino]-2-methylpropyl (2S)-2-amino propanoate trifluoroacetate. Prepared by deprotection of example 98 by an equivalent procedure to that described for example 23. mp 90°C. The ¹H nmr spectrum (d₆-DMSO)(200MHz) had δ: 1.40 (3H, d, CH₃-ala); 1.55 (6H, s, C(CH₃)₂-CH₂-OCO); 4.15 (1H, q, α-CH-ala); 4.35 (1H, d, CH-OCO); 4.55 (1H, d, CH'-OCO); 7.45-7.65 (3H, unresolved, H-2, H-3 and H-4); 7.85 (2H, m, H-6 and H-7); 8.10-8.55 (5H, m, H-5, H-8 and RNH₃^{⊕}); 10.15 (1H, s, ArNH). The electrospray (+)(Cone 20V) mass spectrum had m/z: 367 (100%)(RNH₃^{⊕}). The electrospray (-)(Cone -20V) mass spectrum had m/z: 113 (100%).
Example 100: 2-[(9,10-dioxoanthryl)amino]-2-methylpropyl (2S)-2-[(tert-butoxy)-carbonylamino]-4-methylpentanoate. Prepared from anthraquinone-spacer compound (6) and N-^{t}Boc-L-leucine. The FAB(+) mass spectrum had m/z: 509 (M^{⊕}+1) (73%); 264(100%).
Example 101: 2-[(9,10-dioxoanthryl)amino]-2-methylpropyl (2S)-2-amino-4-methylpentanoate trifluoroacetate. Prepared by deprotection of example 100 by an equivalent procedure to that described for example 23. The electrospray (+)(Cone 20V) mass spectrum had m/z: 409 (100%)(RNH₃^{⊕}); 132 (9%). The electrospray (-)(Cone -20V) mass spectrum had m/z: 113 (100%); 69 (12%).
Example 102: 2-{4-[9,10-dioxoanthryl)amino]phenyl}ethyl (2S)-2-[(tert-butoxy)-carbonylamino]propanoate. Prepared from anthraquinone-spacer compound (7) and N-^{t}Boc-L-alanine. mp 124 °C. The FAB(+) mass spectrum had m/z: 514 (64%)(M^{⊕}+1), 326 (100%). M, 513.
Example 103: 2-{4-[9,10-dioxoanthryl)amino]phenyl}ethyl (2S)-2-aminopropanoate tri-fluoroacetate. Prepared by deprotection of example 102 by an equivalent procedure to that described for example 23. mp 168 °C. The electrospray (+)(Cone 20V) mass spectrum had m/z: 415 (100%)(RNH₃^{⊕}). The electrospray (-)(Cone -20V) mass spectrum had m/z: 113 (38%) 69 (100%).
Example 104: 2-{4-[9,10-dioxoanthryl)amino]phenyl}ethyl (2R)-2-[(tert-butoxy)-carbonylamino]propanoate. Prepared from anthraquinone-spacer compound (7) and N-^{t}Boc-D-alanine. mp 124 °C. The FAB(+) mass spectrum had m/z: 514 (64%)(M^{⊕}+1), 326 (100%). M, 513.
Example 105: 2-{4-[9,10-dioxoanthryl)amino]phenyl}ethyl (2R)-2-aminopropanoate tri-fluoroacetate. Prepared by deprotection of example 104 by an equivalent procedure to that described for example 23. mp 168 °C. The electrospray (+)(Cone 50V) mass spectrum had m/z: 415 (40%)(RNH₃^{⊕}), 326 (100%). The electrospray (-)(Cone -20V) mass spectrum had m/z: 113 (100%).
Example 106: 2-[4-(9,10-dioxoanthryl)piperazinyl]ethyl 2-[(tert-butoxy)carbonylamino]acetate. Prepared from anthraquinone-spacer compound (8) and N-^{t}Boc-gly mp 68 °C. The FAB(+) mass spectrum had m/z: 494 (100%)(M^{⊕}+1). M, 493.
Example 107: 2-[4-(9,10-dioxoanthryl)piperazinyl]ethyl 2-aminoacetate bis-trifluoroacetate. Prepared by deprotection of example 106 by an equivalent procedure to that described for example 23. mp 134 °C
Example 108: 1-[2-(2-{N-tertiarybutoxycarbonyl-L-alanyloxy}ethoxy)ethyl amino]anthracene-9,10-dione. Prepared from anthraquinone-spacer compound (5) and N-^{t}Boc-L-alanine. Low resolution (EI) mass spectrum had m/z: 483 (M^{⊕}+1). M, 482.
Example 109: 1-[2-(2-{L-alanyloxy}ethoxy)ethylamino]anthracene-9,10-dione trifluoroacetate. Prepared by deprotection of example 108 by an equivalent procedure to that described for example 23. The ¹H nmr spectrum (d₆-DMSO)(200MHz) had δ: 1.40 (3H, d, CH₃-ala); 3.50 (2H, t, NH-CH₂-); 3.75 (4H, m, CH₂-O-CH₂); 4.15 (1H, m, α-CH); 4.30 (2H, m, CH₂-OCO); 7.20 (1H, dd, H-2); 7.40 (1H, dd, H-4); 7.55 (1H, t, H-3); 7.80 (2H, m, H-6 and H-7); 8.10 (2H, m, H-5 and H-8); 8.50 (3H, br.s, NH₃^{⊕}); 9.70 (1H, s, ArNH). The electrospray (+) mass spectrum had m/z: 383 (RNH₃^{⊕}).The electrospray (-) mass spectrum had m/z: 113.
Example 110: 1-[2-(2-{N-tertiarybutoxycarbonyl-D-alanyloxy}ethoxy)ethyl amino]anthracene-9,10-dione. Prepared from anthraquinone-spacer compound (5) and N-^{t}Boc-D-alanine. Low resolution (EI) mass spectrum had m/z: 483 (M^{⊕}+1). M, 482.
Example 111: 1-[2-(2-{D-alanyloxy}ethoxy)ethylamino]anthracene-9,10-dione trifluoroacetate. Prepared by deprotection of example 110 by an equivalent procedure to that described for example 23. The ¹H nmr spectrum (d₆-DMSO)(200MHz) had δ: 1.40 (3H, d, CH₃-ala); 3.50 (2H, t, NH-CH₂-); 3.75 (4H, m, CH₂-O-CH₂); 4.15 (1H, m, α-CH); 4.30 (2H, m, CH₂-OCO); 7.20 (1H, dd, H-2); 7.40 (1H, dd, H-4); 7.55 (1H, t, H-3); 7.80 (2H, m, H-6 and H-7); 8.10 (2H, m, H-5 and H-8); 8.50 (3H, br.s, NH₃^{⊕}); 9.70 (1H, s, ArNH). The electrospray (+) mass spectrum had m/z: 383 (RNH₃^{⊕}). The electrospray (-) mass spectrum had m/z: 113.
Example 112: 1-[2-(2-{N-tertiarybutoxycarbonyl-L-phenylalanyloxy} ethoxy) ethylamino]-anthracene-9,10-dione. Prepared from anthraquinone-spacer compound (5) and and N-^{t}Boc-L-phenylalanine. FAB(+) mass spectrum m/z: 559 (M^{⊕}+1). M, 558.
Example 113: 1-[2-(2-{L-phenylalanyloxy}ethoxy)ethylamino]anthracene-9,10-dione trifluoroacetate. Prepared by deprotection of example 112 by an equivalent procedure to that described for example 23. The ¹H nmr spectrum (d₆-DMSO)(200MHz) had δ: 3.05 (2H, m, CH₂-phe); 3.55 (2H, d, NH-CH₂-); 3.70 (4H, m, CH₂-O-CH₂); 4.20-4.40 (3H, unresolved, α-CH and CH₂-OCO); 7.15 (5H, m, C₆H₅); 7.25 (1H, dd, H-2); 7.45 (1H, dd, H-4); 7.65 (1H, t, H-3); 7.85 (2H, m, H-6 and H-7); 8.10 (2H, m, H-5 and H-8); 9.80 (1H, s, ArNH). The electrospray (+) mass spectrum had m/z: 459 (RNH₃^{⊕}). The electrospray(-) mass spectrum had m/z: 113.
Example 114: 1-[2-(2-{N-tertiarybutoxycarbonyl-D-phenylalanyl oxy} ethoxy)ethylamino]-anthracene-9,10-dione. Prepared from anthraquinone-spacer compound (5) and and N-^{t}Boc-D-phenylalanine. The FAB(+) mass spectrum had m/z: 559 (M^{⊕}+1). M, 558.
Example 115: 1-{2-(2-[D-phenylalanyloxy]ethoxy)ethylamino}anthracene-9,10-dione trifluoroacetate. Prepared by deprotection of example 114 by an equivalent procedure to that described for example 23. The ¹H nmr spectrum (d₆-DMSO)(200MHz) had δ: 3.05 (2H, m, CH₂-phe); 3.55 (2H, d, NH-CH₂-); 3.70 (4H, m, CH₂-O-CH₂); 4.20-4.40 (3H, unresolved, α-CH and CH₂-OCO); 7.15 (5H, m, C₆H₅); 7.25 (1H, dd, H-2); 7.45 (1H, dd, H-4); 7.65 (1H, t, H-3); 7.85 (2H, m, H-6 and H-7); 8.10 (2H, m, H-5 and H-8); 9.80 (1H, s, ArNH). The electrospray (+) mass spectrum had m/z: 459 (RNH₃^{⊕}).The electrospray (-) mass spectrum had m/z: 113.
Example 116: 1-(9,10-dioxoanthryl)-4-piperidyl-(2S)-2-[(tert-butoxy)carbonylamino]propanoate. Prepared from anthraquinone-spacer compound (9) and N-^{t}Boc-L-alanine. mp 98 °C. The FAB(+) mass spectrum had m/z: 501 (16%)(M+Na), 479 (100%)(M^{⊕}+1). M, 478.
Example 117: 1-(9,10-dioxoanthryl)-4-piperidyl-(2S)-2-aminopropanoate trifluoroacetate. Prepared by deprotection of example 116 by an equivalent procedure to that described for example 23. mp 102 °C. The ¹H nmr spectrum (d₆-DMSO)(200MHz) had δ: 1.48 (3H, d, CH₃-ala); 1.95 (2H, m, H-3',3"); 2.10 (2H, m, H-5',5"); 3.15 (2H, m, H-2',2"); 3.35 (2H, m, H-6',6"); 4.15 (1H, q, α-CH); 5.05 (1H, quintet, CH-OCO); 7.55 (1H, dd, H-4); 7.75 (2H, m, H-3 and H-2); 7.85 (2H, m, H-6 and H-7); 8.15 (2H, m, H-5 and H-8); 8.40 (3H, br.s, NH₃^{⊕}). The electrospray (+)(Cone 50V) mass spectrum had m/z: 379 (100%)(RNH₃^{⊕}), 290 (50%). The electrospray (-)(Cone - 20V) mass spectrum had m/z: 113 (100%).
Example 118: (2S)-2-[(9,10-dioxoanthryl)amino]propyl (2S)-2-[tert-butoxy)-carbonylamino]propanoate. Prepared from anthraquinone-spacer compound (10) and N-^{t}Boc-L-alanine. mp 127 °C. C₂₅H₂₈N₂O₆ requires C 66.4, H 6.2, N 6.2%. Found C 66.3, H 5.9, N 6.1%. The FAB(+) mass spectrum had m/z: 453 (66%)(M^{⊕}+1) 251 (100%). M, 452.
Example 119: (2S)-2-[(9,10-dioxoanthryl)amino]propyl (2S)-2-aminopropanoate trifluoroacetate. Prepared by deprotection of example 118 by an equivalent procedure to that described for example 23. mp 122 °C. The ¹H nmr spectrum (d₆-DMSO)(200MHz) had δ:1.45 (6H, unresolved, CH₃-ala and CH₃-spacer); 4.05-4.30 (3H, unresolved, NH-CH and CH₂-OCO); 4.40 (1H, q, α-CH-ala); 7.45 (1H, unresolved, H-2 and H-4); 7.70 (1H, t, H-3); 7.90 (2H, m, H-6 and H-7); 8.15 (2H, m, H-5 and H-8); 8.45 (3H, br.s NH₃^{⊕}); 9.0 (1H, d, ArNH).
Example 120: [(2S)-1-(9,10-dioxoanthryl)pyrrolidin-2-yl]methyl (2S)-2-[(tert-butoxy)carbonylamino]propanoate. Prepared from anthraquinone-spacer compound (11) and N-^{t}Boc-L-alanine. mp 110°C. C₂₇H₃₀N₂O₆ requires C 67.8, H 6.3, N 5.9%. Found C 68.3, H 6.0, N 5.8%.
Example 121: [(2S)-1-(9,10-dioxoanthryl)pyrrolidin-2-yl]methyl (2S)-2-aminopropanoate trifluoroacetate. Prepared by deprotection of example 120 by an equivalent procedure to that described for example 23. The ¹H nmr spectrum (d₆-DMSO)(200MHz) had δ: 1.35 (3H, d, CH₃-ala); 1.62 (1H, m, β-CH); 1.95 (2H, m, γ-CH₂); 2.25 (1H, m, β-CH'); 2.40 (1H, m, δ-CH); 3.60 (1H, m, δ-CH'); 4.10 (1H, q, α-CH-ala); 4.30 (2H, m, CH₂-OCO); 4.45 (1H, m, α-CH-pro); 7.65 (3H, s, H-2, H-3 and H-4); 7.85 (2H, m, H-6 and H-7); 8.15 (2H, m, H-5 and H-8); 8.40 (3H, br.s NH₃^{⊕}). The electrospray (+)(Cone 50V) mass spectrum had m/z: 379 (38%)(RNH₃^{⊕}), 308 (100%). The electrospray (-)(Cone -20V) mass spectrum had m/z: 113 (100%). C₂₄H₂₃N₂O₆F₃ requires C 58.5, H 4.7, N 5.7%. Found C 58.4, H 4.4, N 5.6%.
   The following examples [(122)-(125)] were prepared by an equivalent procedure to that described for example (22), in the N-protected form, except that the synthesis began with the appropriate N-tertiarybutoxycarbonyl-protected amino acid in place of N-^{t}Boc-L-ala, and the appropriate anthraquinone spacer compound from examples 17-18, followed by N-deprotection of the first formed ester conjugate using trifluoroacetic acid by analogous procedures to the preparation of example 23.
Example 122: 4-hydroxy-1-[3-(N-tertiarybutoxycarbonyl glycyloxy) propyl amino]anthracene-9,10-dione. Prepared from anthraquinone-spacer compound (17) and N-^{t}Boc-glycine. mp 124 °C. The FAB(+) mass spectrum had m/z: 453 (75%)(M^{⊕}+1) 454 (100%). M, 454.
Example 123: 4-hydroxy-1-[3-(glycyloxy)propylamino]anthracene-9,10-dione trifluoroacetate. Prepared by deprotection of example 122 by an equivalent procedure to that described for example 23. mp 188 °C. The ¹H nmr spectrum (d₆-DMSO)(200MHz) had δ:2.00 (2H, quintet, NH-CH₂-CH₂); 3.55 (2H, q, NH-CH₂); 3.90 (2H, s, CH₂-gly); 4.30 (2H, t, CH₂-OCO); 7.35 (1H, d, H-2); 7.50 (1H, d, H-3); 7.90 (2H, m, H-6 and H-7); 8.20 (2H, m, H-5 and H-8) 10.30 (1H, t, ArNH). The electrospray (+)(Cone 50V) mass spectrum had m/z: 355 (100%)(RNH₃^{⊕}). The electrospray (-)(Cone -20V) mass spectrum had m/z: 113 (100%).
Example 124: 4-hydroxy-1-[3-(N-tertiarybutoxycarbonyl-L-alanyloxy) propyl amino]-anthracene-9,10-dione. Prepared from anthraquinone-spacer compound (17) and N-^{t}Boc-L-alanine. The FAB(+) mass spectrum had m/z: 469 (100%)(M^{⊕}+1).
Example 125: 4-hydroxy-1-[3-(L-alanyloxy)propylamino]anthracene-9,10-dione trifluoroacetate. Prepared by deprotection of example 124 by an equivalent procedure to that described for example 23. Electrospray (+)(Cone 20V) mass spectrum had m/z: 369 (100%)(RNH₃^{⊕}).Electrospray(-)(Cone -20V)mass spectrum had m/z: 113 (100%). The following examples [(126)-(127)] were prepared by an equivalent procedure to that described for example (22), in the N-protected form, except that the synthesis began with the appropriate N-tertiarybutoxycarbonyl-protected amino acid in place of N-^{t}Boc-L-ala, and the appropriate anthraquinone spacer compound from examples 19-20, followed by N-deprotection of the first formed ester conjugate using trifluoroacetic acid by analogous procedures to the preparation of example 23.
Example 126: 4,8-dihydroxy-1-[3-(N-tertiarybutoxycarbonylglycyloxy)propylamino]-anthracene-9,10-dione. Prepared from anmraquinone-spacer compound (19) and N-^{t}Boc-glycine. mp 152 °C. C₂₄H₂₆N₂O₈ requires C 61.3, H 5.6, N 6.0%. Found C 61.6, H 5.5, N 5.9%. The FAB(+) mass spectrum had m/z: 471 (48%)(M^{⊕}+1) 268 (100%). M, 470.
Example 127: 4,8-dihydroxy-1-[3-(glycyloxy)propylamino]anthracene-9,10-dione trifluoroacetate. Prepared by deprotection of example 126 by an equivalent procedure to that described for example 23. mp 196 °C. The ¹H nmr spectrum (d₆-DMSO)(200MHz) had δ:2.00 (2H, quintet, NH-CH₂-CH₂; 3:50 (2H, q, NH-CH₂); 3.80 (2H, s, CH₂-gly); 4.25 (2H, t, CH₂-OCO); 7.25 (2H, m, H-2 and H-3) 7.45 (1H, d, H-7); 7.65 (2H, m, H-5 and H-6); 9.85 (1H, t, ArNH). C₂₁H₁₉N₂O₈F₃ requires C 52.1, H 4.0, N 5.8%. Found C 51.8, H 3.7, N 5.7%. The electrospray (+)(Cone 80V) mass spectrum had m/z: 371 (100%)(RNH₃^{⊕}).

### DIPEPTIDES

The following examples [(128)-(133)] were prepared by an equivalent procedure to that described for example (22), in the N-protected form, except that the synthesis began with the appropriate N-tertiarybutoxycarbonyl-protected dipeptide in place of N-^{t}Boc-L-ala, and the appropriate anthraquinone spacer compound from examples 1-16, followed by N-deprotection of the first formed ester conjugate using trifluoroacetic acid by analogous procedures to the preparation of example 23.
Example 128: 1-[3-(N-tertiarybutoxycarbonyl glycylglycyloxy)propylamino] anthracene-9,10-dione. Prepared from anthraquinone-spacer compound (2) and N-^{t}Boc-glycylglycine. The FAB(+) mass spectrum had m/z: 496 (100%)(M^{⊕}+1); 396 (12%%)
Example 129: 1-[3-(glycylglycyloxy)propylamino]anthracene-9,10-dione trifluoroacetate.
   Prepared by deprotection of example 128 by an equivalent procedure to that described for example 23. The ¹H nmr spectrum (d₆-DMSO)(200MHz) had δ: 2.0 (2H, quintet, CH₂); 3.45 (2H, q, CH₂NH); 3.6 (2H, s, CH₂NH₃); 4.1 (2H, d, CH₂-gly); 4.25 (2H, t, CH₂OCO); 7.25 (1H, dd, H-2); 7.45 (1H, dd, H-4); 7.65 (1H, t, H-3); 7.85 (2H, m, H-6, H-7); 8.0-8.2 (5H, m, unresolved, H-5, H-8 and NH₃^{⊕}); 8.85 (1H, t, NHCO); 9.75 (1H, t, ArNH). Electrospray (+)(Cone 50V) mass spectrum had m/z: 396 (22%) (RNH₃^{⊕});87(100%).Electrospray(-)(Cone-20V)mass spectrum had m/z:113 (100%).
Example 130: 1-[3-(N-tertiarybutoxycarbonylglycyl-L-prolyloxy)propylamino]anthracene-9,10-dione. Prepared from anthraquinone-spacer compound (2) and N-^{t}Boc-glycyl-L-proline. The FAB(+) mass spectrum had m/z: 536 (100%)(M^{⊕}+1).
Example 131: 1-[3-(glycyl-L-prolyloxy)propylamino]anthracene-9,10-dione trifluoroacetate. Prepared by deprotection of example 130 by an equivalent procedure to that described for example 23. mp 146 °C. The ¹H nmr spectrum (d₆-DMSO)(200MHz) had δ: 1.95 (5H, unresolved, β-CH-pro, γ- CH₂-pro and NH-CH₂-CH₂-CH₂); 2.15 (1H, m, β-CH'-pro); 3.45 (4H, unresolved, AQNH-CH₂ and δ- CH₂-pro); 3.80 (2H, s, CH₂-gly); 4.25 (2H, t, CH₂OCO); 4.45 (1H, m, α-CH-pro); 7.25 (1H, dd, H-2); 7.40 (1H, dd,H-4); 7.60 (1H, t, H-3); 7.85 (2H, m, H-6, H-7); 8.10 (2H, m, H-5 and H-8); 9.75 (1H, t, ArNH). Electrospray(+)(Cone50V)mass spectrum m/z: 436 (100%)(RNH₃^{⊕}).Electrospray(-)(Cone-20V)mass spectrum had m/z: 113 (100%).
Example 132: 1-[3-(N-tertiarybutoxycarbonyl-L-leucylglycyloxy)propylamino]anthracene-9,10-dione. Prepared from anthraquinone-spacer compound (2) and N-^{t}Boc-L-leucylglycine. mp 86 °C. C₃₀H₃₇N₃O₇ requires C 65.3, H 6.8, N 7.6%. Found C 65.3, H 6.9, N 7.7%. The FAB(+) mass spectrum had m/z: 553 (45%)(M^{⊕}+1) 236 (100%). M, 552.
Example 133 :1-[3-(L-leucylglycyloxy)propylamino]anthracene-9,10-dione trifluoroacetate.
   Prepared by deprotection of example 132 by an equivalent procedure to that described for example 23. mp 100 °C. The ¹H nmr spectrum (d₆-DMSO)(200MHz) had δ: 0.90 [6H, m, (CH₃)₂-leu]; 1.45-1.85 (3H, unresolved, β-CH₂-leu and γ- CH-leu); 2.00 (2H, quintet, NH-CH₂-CH₂-CH₂); 3.45 (2H, q, AQNH-CH₂); 3.80 (1H, t, α-CH); 4.00 (2H, m, CH₂-gly); 4.25 (2H, t, CH₂OCO); 7.30 (1H, dd, H-2); 7.45 (1H, dd, H-4); 7.65 (1H, t, H-3); 7.85 (2H, m, H-6, H-7); 7.95-8.25 (5H, unresolved, H-5 and H-8 and NH₃^{⊕}).); 8.95 (1H, t, NHCO); 9.70 (1H, t, AQNH). The electrospray (+)(Cone 20V) mass spectrum had m/z: 452 (100%)(RNH₃^{⊕}). The electrospray (-)(Cone -20V) mass spectrum had m/z: 113 (100%).
   The following examples [(134)-(135)] were prepared by an equivalent procedure to that described for example (22), in the N-protected form, except that the synthesis began with the appropriate N-tertiarybutoxycarbonyl-protected dipeptide in place of N-^{t}Boc-L-ala, and the appropriate anthraquinone spacer compound from examples 19-21, followed by N-deprotection of the first formed ester conjugate using trifluoroacetic acid by analogous procedures to the preparation of example 23.
Example 134: (2S)-2-[(4,8-dihydroxy-9,10-dioxoanthryl)amino]-3-phenylpropyl (2S) -1-{2-[(tert-butoxy)carbonylamino]acetyl}pyrrolidine-2-carboxylate. Prepared from anthraquinone-spacer compound (21) and N-^{t}Boc-glycyl-L-proline. mp 100 °C. C₃₅H₃₇N₃O₉ requires C 65.3, H 5.8, N 6.5%. Found C 65.1, H 5.6, N 6.3%. The FAB(+) mass spectrum had m/z: 644 (54%)(M^{⊕}+1) 149 (100%). M, 643.
Example 135: (2S)-2-[(4,8-dihydroxy-9,10-dioxoanthryl)amino]-3-phenylpropyl (2S) -1-(2-aminoacetyl)pyrrolidine-2-carboxylate trifluoroacetate. Prepared by deprotection of example 134 by an equivalent procedure to that described for example 23. mp 135 °C. Electrospray (+)(Cone 50V) mass spectrum had m/z: 544 (100%)(RNH₃^{⊕}). Electrospray (-)(Cone -20V) mass spectrum had m/z: 113 (100%).
   The following examples [(136)-(137)] were prepared by an equivalent procedure to that described for example (22), in the N-protected form, except that the synthesis began with the appropriate N-tertiarybutoxycarbonyl-protected amino acid in place of N-^{t}Boc-L-ala, and the appropriate anthraquinone spacer compound from examples 1-16, followed by N-deprotection of the first formed ester conjugate using trifluoroacetic acid by analogous procedures to the preparation of example 23.
Example 136: 1-[2-(N-tertiarybutoxycarbonyl-L-prolyloxy)ethyl amino] anthracene-9,10-dione. Prepared from anthraquinone-spacer compound (1) and N-^{t}Boc-L-proline. The FAB(+) mass spectrum had m/z: 465 (100%)(M^{⊕}+1).
Example 137: 1-[2-(prolyloxy)ethylamino]anthracene-9,10-dione trifluoroacetate. Prepared by deprotection of example 136 by an equivalent procedure to that described for example 23. The ¹H nmr spectrum (d₆-DMSO)(200MHz) had δ: 1.85 (2H, m, γ-CH₂-pro); 2.05 (1H, m, β-CH-pro); 2.20 (1H, m, β-CH'-pro); 3.20 (2H, m, δ-CH₂-pro); 3.70 (2H, q, AQNH-CH₂); 4.40 (3H, unresolved, α-CH-pro and CH₂OCO); 7.35 (1H, dd, H-2); 7.45 (1H, dd, H-4); 7.65 (1H, t, H-3); 7.85 (2H, m, H-6, H-7); 8.10 (2H, m, H-5 and H-8); 9.75 (1H, t, AQNH). The FAB(+) mass spectrum had m/z: 365 (100%)(RNH₃^{⊕}).

Examples 138 to 140 see Table 2 were prepared by methods analogous to those for 22 and 23 but two equivalents of the appropriate N-protected amino acids were reacted with the appropriate spacer derived from leuco-1,4-dihydroanthraquinone and excess aminoalkanol using Method 3.

### Example 141: Investigation of In Vitro Chemosensitivity

Chinese Hamster Ovary (CHO) cell lines were grown as monolayers in Ham's F-10 nutrient mixture supplemented with 5 % foetal calf serum and 5 % heat-inactivated newborn calf serum. All experiments were performed on cells within 10 passages of each other, during which period phenotypes remained stable. All anthraquinone/amino acid conjugates were dissolved in water and not more than 0.01 % DMSO. Activity was determined by an MTT assay after 24 hours exposure using essentially the method of Plumb *et al* (Plumb, J., Milroy, R and Kaye, S.B., 1989, Effects of the pH dependence of 3-(4,4-dimethylthiazol-2-yl)-2,5-diphenyl tetrazolium bromide formazan absorption on chemosensitivity, *Cancer Research*, **49:** 4435-4440). Characteristics of the cell lines are described in full in Cummiags *et al* 1996, *Biochem*.*Pharmacol*. **52**: 979-990.

**Table 1**

| In Vitro Cytotoxicity of Anthraquinone/Amino Acid Conjugates against Chinese Hamster Ovary (CHO) Cell Lines (IC₅₀µM) including ADR lines. | | | |
|---|---|---|---|
| COMPOUND | CHO-K1 | CHO-ADR-1 | CHO-ADR-r |
| Example 43 | 54.0 | 6.6 | 25.2 |
| Example 45 | 40.5 | 46.3 | 31.5 |

### EXAMPLE 142: In vitro activity against MAC 15A adenocarcinoma.

MAC 15A cells were grown in RPMI 1640 medium supplemented with 10% foetal calf serum containing a 1% antibiotic mixture under standard tissue culture conditions and were maintained at 3TC in a humidified atmosphere of 5% CO₂ in air. Cells were harvested from a stock culture in exponential growth phase and plated in 96-well flat-bottomed plates to achieve a final density of 2 x 10³ cells per well. After 2 hours incubation medium was replaced with either fresh medium containing 0.5% DMSO (control) or medium containing test compound dissolved in DMSO at a concentrations from 10mM to 1nM. Chemosensitivity was assessed using MTT assay by the method of Plumb et al Cancer Research 49 (1989) 4435-4440.

Following 96 hours continuous exposure to drug at 37°C cells were incubated with fresh drug-free medium immediately prior to addition of MTT solution (5mg/ml). Medium and MTT were removed after 4 hours and 150µl of DMSO was added. For each plate the absorbance of the resulting solution was measured at the analytical wavelength 550nm for formazan product, using a Labsystem Mutiskan. IC50 values were obtained from growth curves of drug concentration against % survival and are expressed in µm. Results are shown in Table 2 below.

### EXAMPLE 143: In-vivo activity against MAC15A (S/C).

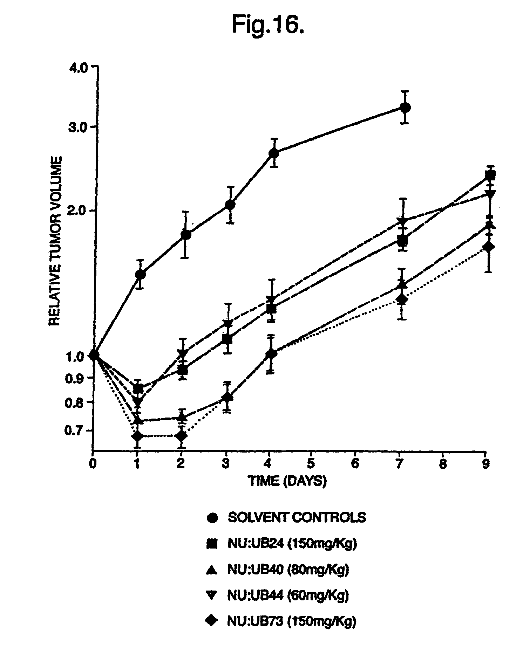

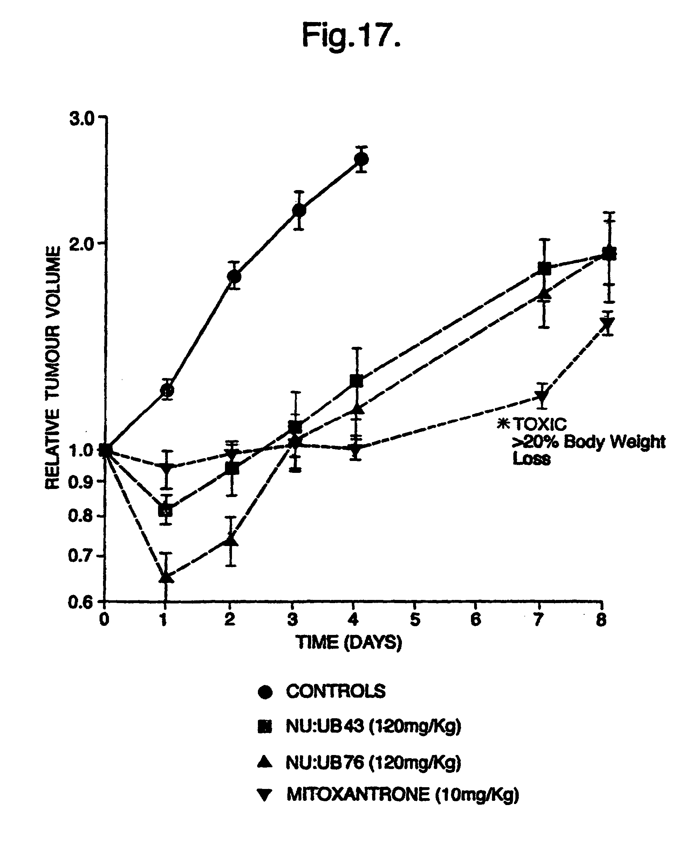

MAC15A cells were obtained from the peritoneum of a donor ascitic mouse. 0.2 ml volumes of the cell suspension were then injected subcutaneously into the lower flank region of each mouse. Approximately 10 mice were set up per treatment or control group. Solid measurable tumours developed after 3 days at which point treatments were commenced (Day 0). Tumours were measured daily using calipers and volumes calculated from the formula (a² x b/2) where a is the smaller diameter and b is the larger. Growth curves were plotted for each group to compare mean relative tumour volume (RTV) against time in days. Statistical analyses were also carried out using the Mann Whitney test which compares the time taken for each tumour to reach RTV x 2 between control and treated animals. Results for Examples 43 and 45 are shown in Figures 16 and 17 below.

### EXAMPLE 144: In vitro Topoisomerase assays.

To determine the effect of the newly synthesised compounds on the catalytic activity of topo I and II (α and β), specific tests measuring relaxation, decatenation and enzyme-mediated cleavage of DNA were employed using purified human topos.

It should be noted that the compounds of this study were assayed against each of the purified α and β isoforms of human topo II. In contrast, the majority of published studies on topo II from human cell lines have concentrated on the α-isoform or a mixture of isoforms.
DNA Topoisomerase I and II(α and β) Relaxation Assays
Topo I Relaxation Assay protocol.
10 x Topo I Relaxation Buffer;
100mM Tris-Hcl (pH 7.5); 500mM KCl; 1mM EDTA; 50 mM MgCl₂;150mg/ml BSA
Loading Buffer (reaction stop)
5% SDS; 0.25mg/mI Bromophenol Blue; 25% Glycerol
10 x TBE Electrophoresis Buffer (500ml)
Tris Base 54.5g; Boric Acid 27.8g; 0.5M EDTA 20ml; DNA 4µl (400ng); Buffer (10 X) 2 µl; Compound1, 10, 25 and 50 µM; Topo I 0.2 µl (2 units); Distilled H₂O to 20 µl total volume.

To eppendorf micro-tubes (0.5ml) the above solutions were added in the following order: Distilled H₂O, DNA, buffer, compound, and mixed by gently tapping the side of the tube being careful not to disperse the reaction contents. The enzyme was pipetted directly onto the side of the tube and the reactions initiated simultaneously by brief centrifugation. The reaction mixture was incubated for 30mins at 37°C following which the reactions were terminated by the addition of 4µl of the loading buffer. The samples were loaded into the wells of a pre-prepared 0.8% agarose gel prepared and immersed in 1xTBE buffer, and the electrophoresis separation of DNA fragments performed. Electrophoresis was carried out until the blue loading buffer had migrated to around 3/4 the length of the gel, typically around 16 hrs at 20volts, or 3-4 hrs at 60 volts. Each gel was then stained for one hour in 50 µg/ml ethidium bromide in 1xTBE buffer, destained for one hour in H₂O, and photographed.
Topo II Relaxation Assay Protocol
10 x Topo II Relaxation Buffer
500 mM Tris-HCl (pH 7.5); 100 mM mgCI₂ ;50mM EDTA; 300 mg/mI BSA; 10 mM DTT; -20°C
DNA 4 µl (400ng); Buffer (10x) 2 µl; ATP 2 µl; KCl (2M) 0.8 µl; Compound 1, 10, 25 and 50 µM; Topo II 5 µl (1-10 µg); Distilled H₂O to 20 µl total volume.

To eppendorf micro-tubes (0.5ml) the above solutions were added in the following order: Distilled H₂O, DNA, buffer, ATP, KCl, compound, and mixed by gently tapping the side of the tube being careful not to disperse the reaction contents. The enzyme was pipetted directly onto the side of the tube and the reactions initiated simultaneously by brief centrifugation. The reaction mixture was incubated for 30 min's, at 37°C following which the reactions were terminated by the addition of 4µl of the loading buffer. The samples were loaded into the wells of a pre-prepared 0.8% agarose gel prepared and immersed in 1 x TBE buffer, and the electrophoresis separation of DNA fragments performed. Electrophoresis was carried out until the blue loading buffer had migrated to around 3/4 the length of the gel, typically around 16 hrs at 20volts, or 3-4 hrs at 60 volts. Each gel was then stained for one hour in 50µg/mI ethidium bromide in 1 x TBE buffer, destained for one hour in H₂O, and photographed.

### Topoisomerase I and II(α and β) cleavage assays

These assays measure the ability of compounds to stimulate topo mediated DNA cleavage.

### Topo I Cleavage Assay Protocol

DNA 4 µl (400ng); Buffer (10 X) 2 µl Compound 1, 10, 25 and 50 µM; Topo I 4 µl (40 units); Distilled H₂O to 20 µl total volume
10% SDS solution 2.2 µl ; 5mg/ml Proteinase K 2.4 µl

To eppendorf micro-tubes (0.5ml) the above solutions were added in the following order: Distilled H₂O, DNA, buffer, compound, and mixed by gently tapping the side of the tube being careful not to disperse the reaction contents. The enzyme was pipetted directly onto the side of the tube and the reactions initiated simultaneously by brief centrifugation. The reaction mixture was incubated for 30 mins at 37°C and the reaction terminated with the addition of 2.2 µl of 10% SDS solution. The tubes were left for at least 30 seconds and 2.4 µl of 5mg/ml Proteinase K solution added. The tubes were incubated for a further 60 minutes followed by the addition of 4 µl of the loading buffer to terminate the reactions.

The samples were loaded into the wells of a pre-prepared 0.8% agarose gel prepared and immersed in 1xTBE buffer, and the electrophoresis separation of DNA fragments performed. Electrophoresis was carried out until the blue loading buffer had migrated to around 3/4 the length of the gel, typically around 16 hrs at 20volts, or 3-4 hrs at 60 volts. Each gel was then stained for one hour in 50 µg/1ml ethidium bromide in 1 x TBE buffer, destained for one hour in H₂O, and photographed.

### Topo II Cleavage Assay Protocol

DNA 4 µl (400ng); Buffer (10X) 2 µl; ATP2 µl (optional depending on the mode of cleavage); KCl (2M) 0.8 µl; Compound 1, 10, 25 and 50 µM; Topo II 5 µl (1-10 µg); Distilled H₂O to 20 µl total volume; 10% SDS solution 2.2 µl; 5mg/ml Proteinase K 2.4 µl

To eppendorf micro-tubes (0.5ml) the above solutions were added in the following order: Distilled H₂O, DNA, buffer, ATP, KCl, compound, and mixed by gently tapping the side of the tube being careful not to disperse the reaction contents. The enzyme was pipetted directly onto the side of the tube and the reactions initiated simultaneously by brief centrifugation. The reaction mixture was incubated for 30 mins at 37°C and the reaction terminated with the addition of 2.2µl of 10% SDS solution. The tubes were left for at least 30 seconds and 2.4 µl of 5mg/ml proteinase K solution added. The tubes were incubated for a further 60 minutes followed by the addition of 4µl of the loading buffer to terminate the reactions.

The samples were loaded into the wells of a pre-prepared 0.8 % agarose gel prepared and immersed in 1 xTBE buffer, and the electrophoresis separation of DNA fragments performed. Electrophoresis was carried out until the blue loading buffer had migrated to around 3/4 the length of the gel typically around 16 hrs at 20 volts, or 3-4 hrs at 60 volts. Each gel was then stained for one hour in 50 µg/ml ethidium bromide in 1 x TBE buffer, destained for one hour in H₂O, and photographed. Results are included in Table 3 below.

### EXAMPLE 146: Topisomerase decatenation assay.

This method uses catenated DNA substrate prepared from the kinetoplast of the insect trypanosome *Crithidia fasciculata*. Upon incubation with topo II, which engages DNA in a double stranded breakage-reunion cycle, DNA minicircles are released. Addition of topo-inhibiting drugs may prevent decatenation.

### Topo II Decatenation Assay Protocol

This assay is the same as the Topo II Relaxation assay except that the pBR 322 Plasmid DNA is replaced with kDNA.
Kinetoplast DNA (kDNA) 4 µl (400ng); Buffer (10X) 2 µl; ATP 2 µl; KCl (2M) 0.8µl; Compound 1,10,25 and 50 µM; Topo II 5 µl (1-10 µg); Distilled H₂O to 20 µl total volume.

To eppendorf micro-tubes (0.5ml) the above solutions were added in the following order: Distilled H₂O, kDNA, buffer, ATP, KCl, compound, and mixed by gently tapping the side of the tube being careful not to disperse the reaction contents.

The enzyme was pipetted directly onto the side of the tube and the reactions initiated simultaneously by brief centrifugation. The reaction mixture was incubated for 30 mins at 37°C and the reaction terminated with the addition of 4µl of loading buffer. The samples were loaded into the wells of a pre-prepared 0.8% agarose gel prepared and immersed in 1x TBE buffer, and the electrophoresis separation of DNA fragments performed. Electrophoresis was carried out until the blue loading buffer had migrated to around % the length of the gel, typically around 16 hrs at 20volts, or 3-4 hrs at 60 volts. Each gel was then stained for one hour in 50 µg/ml ethidium bromide in 1x TBE buffer, destained for one hour in H₂O, and photographed. Results are included in Table 3 below

### EXAMPLE 147: DNA Binding Assay.

The propensity of selected compounds to bind to DNA in the absence of topos was measured in order to identify compounds that would bind weakly, or not at all. Such compounds were thought less likely to exhibit non-specific toxicity or cause chromosomal damage.A topoisomerase I/DNA unwinding assay was used determine binding constants of the compound to DNA by displacement of either ethidium bromide (an intercalator) or Hoechst dye 33258 (a groove binder) which form a DNA-bound fluorescent complex measured by fluorescence spectroscopy.

### DNA Binding Assay Protocol

In order to detect the strength and mode of anthraquinone compound binding to DNA the displacement of known DNA binders was detected by measuring the fluorescence of a DNA/fluorescent compound complex. The addition of known concentrations of ethidium bromide, an interchelator, and Hoescht Dye, a groove binder, cause a fluorescent DNA/binder complex that can be detected and the fluorescence quantified. The addition of anthraquinone compounds displaces the interchelators or groove binders, depending on the compound mode of DNA binding, and therefore reduces the fluorescence accordingly. The preferred binding action of compounds can be quantified by determining the reduction in fluorescence resulting from a given concentration of compound. The quantification of a compounds ability to bind to DNA is expressed, as the concentration required to displace 50% of the ethidium bromide or Hoescht Dye, thus reducing the fluorescence by 50%. Therefore, the values produced are QE₅₀ for ethidium, bromide, or QH₅₀ for Hoescht Dye.
Compound: 300, 60, 10 and 10/6gM (1.66µM) solution concentrations were prepared for use in the assay to produce a range of compound. Concentrations. 100, 200 and 300 µl of each dilution were used in the assay reaction. This provides a range of concentrations: - 30, 20, 10, 6, 4, 2, 1, 0.67, 0.33, 0.17, 0.11, 0.06, 0.00 µM in the assay environment. Intermediate concentrations can be utilised to enhance the accuracy of the displacement concentration determination.
Buffer: 100mM Tris, NaCI 500mM. DNA: Calf thymus sodium salt. Stock solution of 200 µM was prepared in 10X assay buffer. Dilution of DNA therefore provided the correct concentration of assay buffer in the assay cell.300µg DNA was used in 3ml reaction mixture therefore 20 µM DNA concentration. Hoescht Dye 2 µM final concentration, therefore 100 µl of a 60 µM stock solution was added to a 3ml assay.
Ethidium Bromide 2 µM final concentration therefore 100 µl of a 60 µM stock solution was added to a 3ml assay. Distilled H₂O Water was added to produce 3ml reaction volume. Order of addition:1. DNA, 2. Water, 3. Drug, 4. Dye

The assay was completed with both Hoescht Dye and Ethidium. Bromide for each compound. The ethidium bromide is a DNA inter-chelator, Hoescht Dye is a minor groove binder. 100, 200 and 30 µg of the lowest compound dilution was assayed. This procedure was repeated with all further dilution's. This provided a curve of fluorescence intensity decrease with increasing compound concentration. QE₅₀ and QH₅₀ values were determined by extrapolating the concentration of compound at the point where the fluorescence intensity was reduced by 50%. Controls involving compound only and ethidium. bromide or Hoechst Dye without compound were carried out for each experiment. Two readings for each concentration of compound were performed per experiment, each experiment was repeated at least three times. Fluorometer: Perkin Elmer Luminescence Spectrometer LS 50B.

### Spectrometer settings for Ethidium Bromide:

| | | |
|---|---|---|
| FROM | TO | EXCITATION |
| 570nm | 630nm | 546 |
| SCAN SPEED | EX SLIT | EM SLIT |
| 200 | 10 | 15 |

### Settings for Hoechst Dye:

| | | |
|---|---|---|
| FROM | TO | EXCITATION |
| 440nm | 490nM | 353 |
| SCAN SPEED | EX SLIT | EM SLIT |
| 200 | 15 | 2.5 |

### Results are shown in table 4 below

**TABLE 2:**

| ***IN VITRO* CHEMOSENSITIVITY OF SPACER-LINKED ANTHRAQUINONE PEPTIDE CONJUGATES AGAINST MAC 15A ADENOCARCINOMA OF THE COLON** | | | | |
|---|---|---|---|---|
| EXAMPLE | NU:UB CODE | Amino-R⁷-O-SPACER TYPE (Anthraquinone sub) | PEPTIDE MOTIF | IC₅₀ µM |
| 23 | 90 | -NH-ETHOXY | Ala-TFA | 7.5 |
| 25 | 87 | -NH-ETHOXY | Val-TFA | 30.0 |
| 27 | 91 | -NH-ETHOXY | D-Ala-TFA | 14.0 |
| 29 | 108 | -NH-PROPOXY | Ala-TFA | 9.7 |
| 35 | 82 | -NH-PROPOXY | Val-TFA | 2.9 |
| 39 | 71 | -NH-PROPOXY | Phe-TFA | 3.0 |
| 43 | 73 | -NH-BUTOXY | Ala-TFA | 2.6 |
| 45 | 76 | -NH-BUTOXY | D-Ala-TFA | 2.9 |
| 51 | 103 | -NH-PROPOXY(AQ-4-OH) | Val-TFA | 4.9 |
| 53 | 104 | -NH-BUTOXY(AQ-4-OH) | Val-TFA | 5.3 |
| 57 | 100 | -NH-PROPOXY(AQ-4,8-di-OH) | Val-TFA | 3.8 |
| 59 | 117 | -NH-ETHOXY | Gly-TFA | 3.5 |
| 61 | 109 | -NH-PROPOXY | Gly-TFA | 10.6 |
| 63 | 107 | -NH-PROPOXY | D-Ala-TFA | 6.9 |
| 65 | 162 | -NH-PROPOXY | D-Phe | 4.8 |
| 67 | 111 | -NH-PROPOXY | Pro-TFA | 8.3 |
| 69 | 112 | -NH-PROPOXY | D-Pro-TFA | 10.2 |
| 71 | 120 | -NH-PROPOXY | Orn(δ-Z)-α-TFA | 2.4 |
| 73 | 160 | -NH-PROPOXY | Phg-TFA | 5.0 |
| 75 | 161 | -NH-PROPOXY | D-Phg-TFA | 4.2 |
| 77 | 172 | -NH-PENTOXY | Trp-TFA | 5.5 |
| 79 | 173 | -NH-PROPOXY | Tyr-(O-Me)-TFA | 3.5 |
| 81 | 110 | -NH-BUTOXY | Gly-TFA | 9.4 |
| 83 | 163 | -NH-BUTOXY | Sar-TFA | 7.2 |
| 85 | 169 | -NH-BUTOXY | Aib-TFA | 3.2 |
| 87 | 167 | -NH-BUTOXY | Abu-TFA | 3.0 |
| 89 | 168 | -NH-BUTOXY | γ-Abu-TFA | 4.5 |
| 91 | 166 | -NH-BUTOXY | Me-Ala-TFA | 4.8 |
| 93 | 125 | -NH-PENTOXY | Ala-TFA | 3.5 |
| 95 | 126 | -NH-PENTOXY | D-Ala-TFA | 3.1 |
| 97 | 119 | -NH-C(CH₃)₂-CH₂O- | Gly-TFA | 15.0 |
| 99 | 128 | -NH-C(CH₃)₂-CH₂O- | Ala-TFA | 35.0 |
| 103 | 156 | 4-(2-hydroxyethyl)phenylamino | Ala-TFA | 4.7 |
| 105 | 157 | 4-(2-hydroxyethyl)phenylamino | D-Ala-TFA | 3.4 |
| 107 | 130 | -(PIPERAZINYL)ETHOXY- | Gly-bis-TFA | 24.0 |
| 109 | 122 | -NH-ETH-O-ETHOXY- | Ala-TFA | 11.5 |
| 111 | 122 | -NH-ETH-O-ETHOXY- | D-Ala-TFA | 24.0 |
| 113 | 123 | -NH-ETH-O-ETHOXY- | Phe-TFA | 7.0 |
| 115 | 124 | -NH-ETH-O-ETHOXY- | D-Phe-TFA | 12.0 |
| 117 | 158 | -4-PIPERIDINOXY- | Ala-TFA | 14.0 |
| 119 | 170 | -L-ALANINOL- | Ala-TFA | 5.5 |
| 121 | 171 | -L-PROLINOL- | Ala-TFA | 22 |
| 123 | 165 | -NH-PROPOXY(AQ-4-OH-) | Gly-TFA | 0.3 |
| 127 | 129 | -NH-PROPOXY(AQ-4,8-di-OH)- | Gly-TFA | 1.3 |
| 129 | 175 | -NH-PROPOXY- | Gly-Gly-TFA | 4.0 |
| 131 | 116 | -NH-PROPOXY- | Pto-Gly-TFA | 2.5 |
| 133 | 127 | PROPOXY | Gly-Leu-TFA | 1.2 |
| 135 | 159 | -L-PHENYLALANINOL-(AQ4-4,8-di-OH)- | Pro-Gly-TFA | 2.5 |
| 137 | 115 | -NH-ETHOXY- | Pro-TFA | 33.5 |
| 138 | 52 | (1,4-BIS)-NH-PROPOXY- | BisAla-TFA | 16.0 |
| 139 | 53 | (1,4-BIS)-NH-ETHOXY- | BisAla-TFA | 44.0 |
| 140 | 57 | (1,4-BIS)-NH-PROPOXY- | BisTyrOBzl TFA | 12.0 |

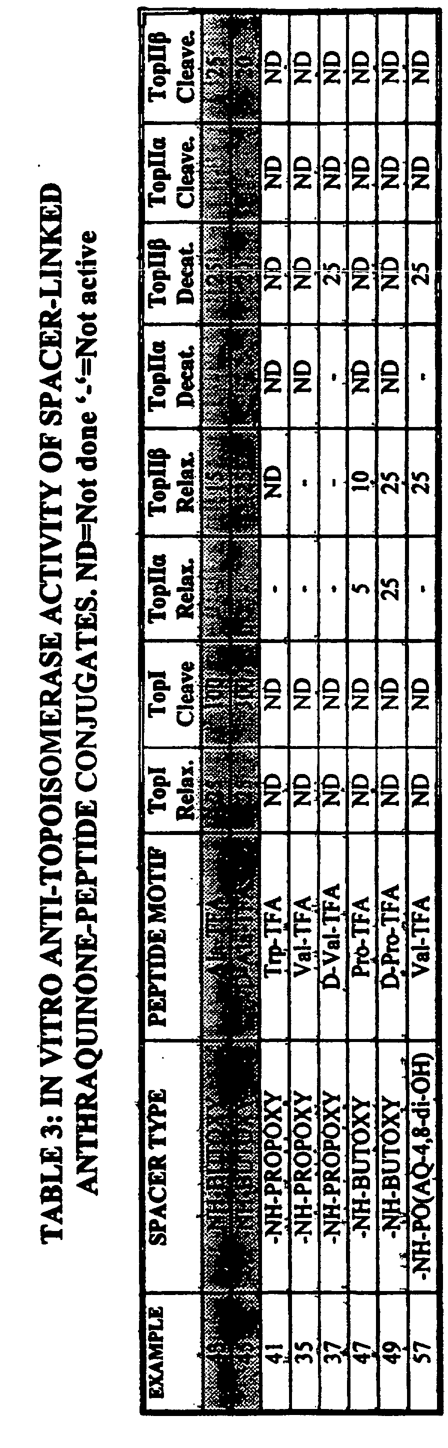

**TABLE 4:**

| **DNA Binding Assay NB=Not binding** | | |
|---|---|---|
| **NU:UB Compound Example** | **QE**_{**50**}**(µM) Intercalating** | **QH**_{**50**}**(µM) Minor groove binding** |
| (Amide)31 | 3.5±0.9 | 2.1±0.4 |
| (Amide)51 | 1.3±0.4 | 1.1±0.2 |
| 73 Ex 43 | NB | NB |
| 76 Ex 45 | NB | NB |
| 89 Ex 33 | NB | NB |
| 104 Ex 53 | 2.9 | 4.4 |
| 107 Ex 63 | NB | NB |
| 108 Ex 29 | NB | NB |
| 111 Ex67 | NB | NB |
| 112 Ex 69 | NB | NB |
| 115 Ex 137 | NB | NB |
| Mitoxantrone | 0.8±0.3 | 1.2±0.3 |

### SUMMARY OF RESULTS:

Results in table 4 show that DNA binding of almost all ester compounds reported on is not measurable using the assay provided as compared activities for all the corresponding amide linked compounds of US 5,733,880 and Mitoxantrone. Compounds demonstrate non-cross resistant activity against resistant cell lines (see Table 1), have broad spectrum activity and shrink MAC15A tumours *in vivo* in test animals. Comparison of Examples 43 and 45, for which most data is available, show this new class of compound to generally be less active against Topoisomerase IIα in *in vitro* assays, active against Topisomerase I and IIβ. Example 43 produces only 20% nicked DNA at 100µM as compared to campothecin which gives 90% nicked plasmid at 10 µM. Furthermore, initial screening in the NCI screen shows compounds of the invention to be cytotoxic against NCI -H460 (lung), MCF7 (breast) and SF-268 (CNS) tumour lines.

It should be noted that while many of the results presented relate to trifluoroacetic acid salts, corresponding salts, eg. acetates, have been found to have equivalent activities.

## Claims

1. A compound having the formula: 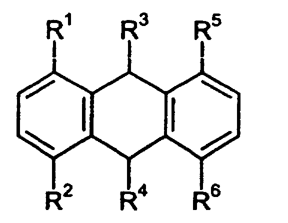 wherein
R¹ and R² are independently hydrogen, hydroxy, alkoxy or acyloxy;
R³ and R⁴ are independently oxo, hydroxy or hydrogen;
one of R⁵ or R⁶ is -A-B and the other is hydrogen, hydroxy, alkoxy, acyloxy, a group -A-B or a group -amino-R⁷-O-Y;
the or each A is independently a spacer group having the formula -amino-R⁷-O- which is bonded to the anthracene ring via the amino group nitrogen and to B via the -O- atom through an ester bond;
R⁷ is a divalent organic radical;
B is an amino acid residue, a peptide group or isostere thereof; and
Y is hydrogen or a capping group,
or a physiologically acceptable derivative thereof.

2. A compound of claim 1, being of Formula II 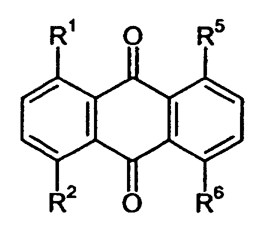 wherein R¹, R², R⁵ and R⁶ are as defined in Claim 1.

3. A compound as claimed in Claim 1 or Claim 2 **characterised in that** one of R⁵ or R⁶ is a group -A-B and the other is hydrogen or hydroxy.

4. A compound as claimed in any one of Claims 1 to 3 **characterised in that** amino, as used with respect to -amino-R⁷-O-, is a group selected from -NH-, -NR¹⁰-or -N=R¹¹- wherein
R¹⁰ is selected from alkyl, alkenyl, aralkyl or aryl and
R¹¹ consists of a moiety with which the -N= makes up a heterocylic ring system containing the nitrogen atom of the aforesaid -N= moiety and up to 6 other members selected from nitrogen, oxygen, sulphur and carbon.

5. A compound as claimed in Claim 4 **characterised in that** 'amino' is a group -N=R¹¹- wherein R¹¹ is a moiety with which -N= makes up an NC₄, NC₅, N₂C₃ or N₂C₄ ring.

6. A compound as claimed in Claim 5 **characterised in that** the ring is selected from pyrrole, 2H-pyrrole, pyrrolidine, pyrroline, imidazole, imidazolidine, imidazoline, pyrazole, pyrazolidine, pyrazoline, pyridine, pyrazine, piperidine, and piperazine.

7. A compound as claimed in any one of Claims 1 to 6 **characterised in that** R⁷ is a divalent group that spaces the moiety -O- from the amino group on the anthracene ring by a contiguous chain of 1 to 20 atoms.

8. A compound as claimed in any one of Claims 1 to 7 **characterised in that** R⁷ is or comprises an alkylene radical having the formula -(CHR)ₙ- where n is an integer and the or each R is independently hydrogen or an alkyl group and n is an integer of 1 to 20.

9. A compound as claimed in any one of Claims 1 to 8 **characterised in that** R⁷ comprises an alkylene radical the carbon chain of which is interrupted by one or more olefinic bonds, heteroatoms, carbocylic and/or heterocyclic rings.

10. A compound as claimed in any one of Claims 1 to 9 wherein B is a residue of an α-amino acid or a peptide group made from α-amino acids.

11. A compound as claimed in any one of Claims 1 to 10 wherein R¹ and R² are both H, R³ and R⁴ are both oxo, R⁵ is a group -A-B and R⁶ is H.

12. A compound as claimed in any one of Claims 1 to 10 wherein R¹ is OH, R² is H, R³ and R⁴ are both oxo, R⁵ is a group -A-B and R⁶ is OH.

13. A compound as claimed in any one of Claims 1 to 10 wherein R¹ and R² are both H, R³ and R⁴ are both oxo, R⁵ is a group -A-B and R⁶ is OH.

14. A compound as claimed in any one of Claims 1 to 6 wherein R¹ and R² are both OH, R³ and R⁴ are both oxo, R⁵ is a group -A-B and R⁶ is OH.

15. A pharmaceutical preparation comprising a pharmaceutically acceptable carrier and/or excipient and a compound as claimed in any one of Claims 1 to 14.

16. A compound or preparation as claimed in any one of Claims 1 to 15 for use in therapy.

17. The use of a compound or preparation as claimed in any one of Claims 1 to 15 in the manufacture of a medicament for treating cancer, viral disease or parasites in humans or animals.

18. A method for the manufacture of a compound as claimed in any one of Claims 1 to 14 **characterised in that** it comprises
(A) reacting a compound of Formula IV: 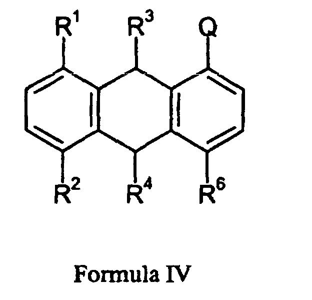 where R¹ to R⁴ are as defined above, R⁶ is hydrogen or hydroxyl and Q is a reactive group such as -Cl, -Br or -OH with an amino alcohol to form a compound having the formula V: 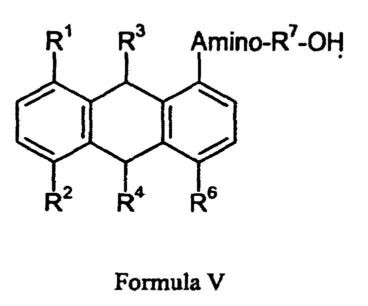 and
(B) reacting the compound of Formula V with an amino acid or peptide or isostere to give a compound of Formula I:
R¹ to R⁶ as defined as

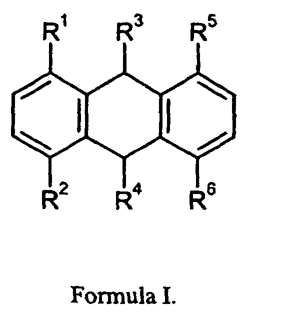

## Patentansprüche

1. Eine Verbindung mit der Formel: 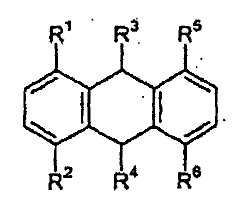 worin
R¹ und R² unabhängig voneinander für Wasserstoff, Hydroxy, Alkoxy oder Acyloxy stehen;
R³ und R⁴ unabhängig voneinander für Oxo, Hydroxy oder Wasserstoff stehen;
eines von R⁵ oder R⁶ für -A-B steht und das andere für Wasserstoff, Hydroxy, Alkoxy, Acyloxy, eine Gruppe -A-B oder eine Gruppe -Amino-R⁷-O-Y steht;
das oder jedes A unabhängig voneinander eine Spacergruppe mit der Formel -Amino-R⁷-O- darstellt, die über den Aminogruppenstickstoff an den Anthracenring und über das -O-Atom durch eine Esterbindung an B gebunden ist;
R⁷ für einen divalenten organischen Rest steht;
B für einen Aminosäurerest, eine Peptidgruppe oder ein Isoster davon steht; und
Y für Wasserstoff oder eine Verkappungsgruppe steht,
oder ein physiologisch verträgliches Derivat davon.

2. Eine Verbindung gemäß Anspruch 1, die die Formel II aufweist: 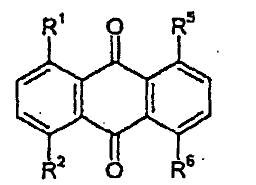 worin R¹, R², R⁵ und R⁶ wie in Anspruch 1 definiert sind.

3. Eine Verbindung gemäß Anspruch 1 oder 2, **dadurch gekennzeichnet, dass** eines von R⁵ oder R⁶ für eine Gruppe -A-B und das andere für Wasserstoff oder Hydroxy steht.

4. Eine Verbindung gemäß einem der Ansprüche 1 bis 3, **dadurch gekennzeichnet, dass** Amino, wie es in Bezug auf -Amino-R⁷-O- angewendet wird, für eine Gruppe steht, die aus -NH-, -NR¹⁰- oder -N=R¹¹- ausgewählt ist, worin
R¹⁰ aus Alkyl, Alkenyl, Aralkyl oder Aryl ausgewählt ist und
R¹¹ aus einem Rest besteht, mit welchem das -N= ein heterocyclisches Ring- system bildet, das das Stickstoffatom des vorstehend genannten -N= Teils und bis zu 6 weitere Glieder, die aus Stickstoff, Sauerstoff, Schwefel und Kohlenstoff ausgewählt sind, enthält.

5. Eine Verbindung gemäß Anspruch 4, **dadurch gekennzeichnet, dass** "Amino" für eine Gruppe -N=R¹¹- steht, worin R¹¹ einen Rest darstellt, mit dem -N= einen NC₄-, NC₅-, N₂C₃- oder N₂C₄-Ring bildet.

6. Eine Verbindung gemäß Anspruch 5, **dadurch gekennzeichnet, dass** der Ring aus Pyrrol, 2H-Pyrrol, Pyrrolidin, Pyrrolin, Imidazol, Imidazolidin, Imidazolin, Pyrazol, Pyrazolidin, Pyrazolin, Pyridin, Pyrazin, Piperidin und Piperazin ausgewählt ist.

7. Eine Verbindung gemäß einem der Ansprüche 1 bis 6, **dadurch gekennzeichnet, dass** R⁷ für eine divalente Gruppe steht, die den Molekültell -O- von der Aminogruppe auf dem Anthracenring mittels einer zusammenhängenden Kette mit 1-20 Atomen beabstandet.

8. Eine Verbindung gemäß einem der Ansprüche 1 bis 7, **dadurch gekennzeichnet, dass** R⁷ ein Alkylenrest ist oder umfasst, der die Formel -(CHR)ₙ- aufweist, worin n eine ganze Zahl darstellt und das oder jedes R unabhängig voneinander für Wasserstoff oder eine Alkylgruppe steht und n eine ganze Zahl von 1-20 ist.

9. Eine Verbindung gemäß einem der Ansprüche 1 bis 8, **dadurch gekennzeichnet, dass** R⁷ einen Alkylenrest umfasst, dessen Kohlenstoffkette durch eine oder mehrere olefinische Bindungen, Heteroatome, carbocyclische und/oder heterocyclische Ringe unterbrochen ist.

10. Eine Verbindung gemäß einem der Ansprüche 1 bis 9, worin B für einen Rest einer α-Aminosäure oder einer Peptidgruppe aus α-Aminosäuren steht.

11. Eine Verbindung gemäß einem der Ansprüche 1 bis 10, worin R¹ und R² beide für H stehen, R³ und R⁴ beide für Oxo stehen, R⁵ für eine Gruppe -A-B steht und R⁶ für H steht.

12. Eine Verbindung gemäß einem der Ansprüche 1 bis 10, worin R¹ für OH steht, R² für H steht, R³ und R⁴ beide für Oxo stehen, R⁵ für eine Gruppe -A-B steht und R⁶ für OH steht.

13. Eine Verbindung gemäß einem der Ansprüche 1 bis 10, worin R¹ und R² beide für H stehen, R³ und R⁴ beide für Oxo stehen, R⁵ für eine Gruppe -A-B steht und R⁶ für OH steht.

14. Eine Verbindung gemäß einem der Ansprüche 1 bis 6, worin R¹ und R² beide für OH stehen, R³ und R⁴ beide für Oxo stehen, R⁵ für eine Gruppe -A-B steht und R⁶ für OH steht.

15. Eine pharmazeutische Zubereitung umfassend einen pharmazeutisch verträglichen Carrier und/oder Arzneimittelträger und eine Verbindung gemäß einem der Ansprüche 1 bis 14.

16. Eine Verbindung oder Zubereitung gemäß einem der Ansprüche 1 bis 15 zur therapeutischen Verwendung.

17. Die Verwendung einer Verbindung oder einer Zubereitung gemäß einem der Ansprüche 1 bis 15 zur Herstellung eines Arzneimittels zur Behandlung von Krebs, Viruserkrankung oder Parasiten in Menschen oder Tieren.

18. Ein Verfahren zur Herstellung einer Verbindung gemäß einem der Ansprüche 1 bis 14, **dadurch gekennzeichnet, dass** es folgendes umfasst:
(A) Umsetzen einer Verbindung der Formel IV: 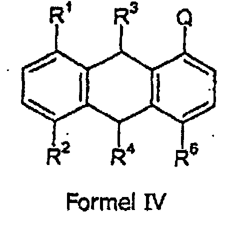 worin R¹ bis R⁴ wie vorstehend definiert sind, R⁶ für Wasserstoff oder Hydroxyl steht und Q für eine reaktive Gruppe wie -Cl, -Br oder -OH steht, mit einem Aminoalkohol, um eine Verbindung mit der folgenden Formel V zu bilden: 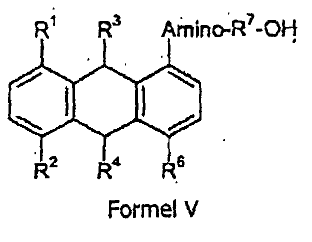 und
(B) Umsetzen der Verbindung gemäß Formel V mit einer Aminosäure oder einem Peptid oder einem Isoster, um eine Verbindung der Formel I zu bilden: 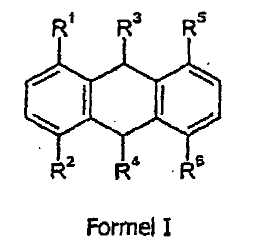
worin R¹ bis R⁶ wie vorstehend definiert sind.

## Revendications

1. Composé ayant la formule : 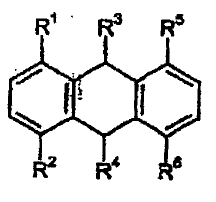 dans laquelle
R¹ et R² sont indépendamment un hydrogène, hydroxy, alcoxy ou acyloxy ;
R³ et R⁴ sont indépendamment oxo, hydroxy ou hydrogène ;
l'un de R⁵ ou R⁶ est -A-B et l'autre est un hydrogène, hydroxy, alcoxy, acyloxy, un groupe -A-B ou un groupe -amino-R⁷-O-Y ;
le ou chaque A est indépendamment un groupe espaceur ayant la formule -amino-R⁷-O- qui est lié au cycle anthracène via l'azote du groupe amino et à B via l'atome -O- à travers une liaison ester ;
R⁷ est un radical organique divalent ;
B est un résidu aminoacide, un groupe peptide ou un isostère de celui-ci ; et
Y est un hydrogène ou un groupe coiffant,
ou un dérivé physiologiquement acceptable de celui-ci.

2. Composé selon la revendication 1, étant de formule II 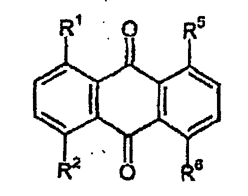 dans laquelle R¹, R², R⁵ et R⁶ sont tels que définis dans la revendication 1.

3. Composé selon la revendication 1 ou la revendication 2 **caractérisé en ce que** l'un de R⁵ ou R⁶ est un groupe -A-B et l'autre est un hydrogène ou hydroxy.

4. Composé selon l'une quelconque des revendications 1 à 3 **caractérisé en ce que** le groupe amino, tel qu'utilisé dans -amino-R⁷-O-, est un groupe choisi parmi -NH-, -NR¹⁰- ou -N=R¹¹- dans lequel
R¹⁰ est choisi parmi un alkyle, alcényle, aralkyle ou aryle et
R¹¹ consiste en un fragment avec lequel le -N= forme un système cyclique hétérocyclique c ontenant l'atome d'azote du fragment -N= précédemment indiqué et jusqu'à 6 autres éléments choisis parmi azote, oxygène, soufre et carbone.

5. Composé selon la revendication 4 **caractérisé en ce que** « amino » est un groupe -N=R¹¹- dans lequel R¹¹ est un fragment avec lequel -N= forme un cycle NC₄, NC₅, N₂C₃ ou N₂C₄.

6. Composé s elon 1 a r evendication 5 c aractérisé en ce que le cycle est choisi parmi le pyrrole, le 2H-pyrrole, la pyrrolidine, la pyrroline, l'imidazole, l'imidazolidine, l'imidazoline, le pyrazole, la pyrazolidine, la pyrazoline, la pyridine, la pyrazine, la pipéridine et la pipérazine.

7. Composé selon l'une quelconque des revendications 1 à 6 **caractérisé en ce que** R⁷ est un groupe divalent qui espace le fragment -O- du groupe amino sur le cycle anthracène par une chaîne contiguë de 1 à 20 atomes.

8. Composé selon l'une quelconque des revendications 1 à 7 **caractérisé en ce que** R⁷ est, ou comprend un radical alkylène ayant la formule -(CHR)ₙ- où n est un entier et le ou chaque R est indépendamment un hydrogène ou un groupe alkyle et n est un entier de 1 à 20.

9. Composé selon l'une quelconque des revendications 1 à 8 **caractérisé en ce que** R⁷ comprend un radical alkylène dont la chaîne carbonée est interrompue par une ou plusieurs liaisons oléfiniques, un ou plusieurs hétéroatomes, cycles carbocycliques et/ou hétérocycliques.

10. Composé selon l'une quelconque des revendications 1 à 9 dans lequel B est un résidu d'un α-aminoacide ou un groupe peptide fait d'α-aminoacides.

11. Composé selon l'une quelconque des revendications 1 à 10 dans lequel R¹ et R² sont tous deux H, R³ et R⁴ sont tous deux oxo, R⁵ est un groupe -A-B et R⁶ est H.

12. Composé selon l'une quelconque des revendications 1 à 10 dans lequel R¹ est OH, R² est H, R³ et R⁴ sont tous deux oxo, R⁵ est un groupe -A-B et R⁶ est OH.

13. Composé selon l'une quelconque des revendications 1 à 10 dans lequel R¹ et R² sont tous deux H, R³ et R⁴ sont tous deux oxo, R⁵ est un groupe -A-B et R⁶ est OH.

14. Composé selon l'une quelconque des revendications 1 à 6 dans lequel R¹ et R² sont tous deux OH, R³ et R⁴ sont tous deux oxo, R⁵ est un groupe -A-B et R⁶ est OH.

15. Préparation pharmaceutique comprenant un support et/ou excipient pharmaceutiquement acceptable et un composé selon l'une quelconque des revendications 1 à 14.

16. Composé ou préparation selon l'une quelconque des revendications 1 à 15 pour utilisation en thérapie.

17. Utilisation d'un composé ou préparation selon l'une quelconque des revendications 1 à 15 dans la fabrication d'un médicament pour traiter le cancer, une maladie virale ou des parasites chez des humains ou des animaux.

18. Procédé de fabrication d'un composé selon l'une quelconque des revendications 1 à 14 **caractérisé en ce qu'**il comprend les opérations consistant à
(A) mettre à réagir un composé de formule IV : 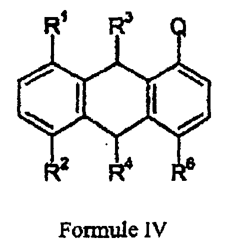 dans laquelle R¹ à R⁴ sont tels que définis ci-dessus, R⁶ est un hydrogène ou hydroxyle et Q est un groupe réactif tel que -Cl, -Br ou -OH avec un aminoalcool pour former un composé ayant la formule V : 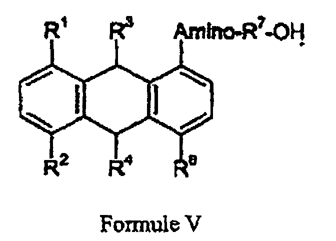 et
(B) mettre à réagir le composé de formule V avec un aminoacide ou un peptide ou un isostère pour donner un composé de formule I : 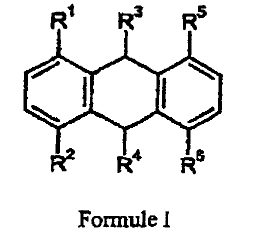
dans laquelle R¹ à R⁶ sont tels que définis ci-dessus.
